# EUROPEAN PATENT APPLICATION

(11) **EP 3 725 287 A1**
(43) Date of publication of application: **21.10.2020**
(21) Application number: 19170311.5
(22) Date of filing: 18.04.2019
(51) Int. Cl.: A61K 6/00, C08F 4/40

(54) **REDOX CURING DENTAL COMPOSITION**

(71) Applicant: Dentsply DeTrey GmbH, 78467 Konstanz (DE)
(72) Inventor: Szillat, Florian, 78462 Konstanz (DE); Renn, Caroline, 78224 Singen (DE); Lalevée, Jacques, 68200 Mulhouse (FR)
(74) Representative: Dietz, Mirko

(57) **Abstract**

A redox curing dental composition comprising:
(a) one or more compounds having a radical polymerizable carbon-carbon double bond;
(b) a redox initiator system comprising
(i) a reducing agent which is a compound of the following formula (I): 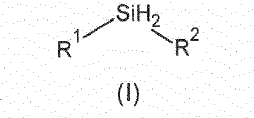 wherein
R¹ and R²
which may be the same or different, independently represent a C₁₋₁₈ hydrocarbyl group, or R¹ and R² form together with the silicon atom to which they are bonded a 3 to 8-membered silicon-containing alicyclic ring, which groups R¹ and/or R² may be substituted with one to three substituents selected from the group consisting of a C₁₋₆ alkoxy group, a halogen atom, or a carboxylic acid group; and
(ii) an oxidizing agent adapted to oxidize the reducing agent of formula (I) in the absence of light for generating a radical capable of initiating radical polymerization of the one or more compounds having a radical polymerizable carbon-carbon double bond.

## Description

### Field of the invention

The present invention relates to a redox curing dental composition comprising one or more compounds having a radical polymerizable carbon-carbon double bond and a redox initiator system containing a specific silane reducing agent and an oxidizing agent adapted to generate radicals by interaction with the reducing agent in the absence of light. Furthermore, the present invention relates to a redox initiator system containing the specific silane reducing agent and the oxidizing agent. Finally, the present invention relates to the redox initiator system for use in the treatment or prevention of endodontic disease, in particular by root canal therapy.

A redox curing dental composition according to the present invention may be provided as a two-pack dental composition having high storage stability even when formulated as aqueous pastes, while at the same time providing high reactivity when the redox initiating system is activated by mixing the reducing agent and the oxidizing agent, and optionally by additional irradiation of light in the presence of a photosensitizer.

### Background of the Invention

Polymerizable dental compositions containing compounds having radical polymerizable carbon-carbon double bonds are known, and further require an initiator system for curing. The initiator system may be a self-curing redox initiator system activated when the components of the redox initiator system are mixed and allowed to react. Alternatively or additionally, the initiator system may be a photoinitiator system which is activated by irradiation, preferably with visible light in the range of from 400 to 800 nm.

In case of dental applications wherein at least a portion of the dental composition is shielded from any subsequent irradiation of light such as in many instances of applying a resin-modified dental cement, a pulp capping composition, or a bonding agent, activation by light may difficult so that the self-curing redox initiator system must provide sufficient curing rate and conversion for curing the radical polymerizable components of the dental composition. Some conventional dental luting cements are even formulated entirely as dark-cure materials. A great effort is documented by the prior art, which is directed to the development of polymerizable redox curing dental compositions having improved properties with regard to physical properties, biocompatibility, aesthetics and handling properties. Redox initiator systems for a redox curing dental composition contain an oxidizing agent and a reducing agent as essential components. Strong oxidizing agents such as peroxides are frequently combined with aromatic tertiary amines as reducing agents in redox curing dental compositions. However, the use of peroxides and/or aromatic amines gives rise to toxicological concerns. Moreover, the presence of amines in acrylate-containing compositions can cause yellowing of the resulting cured composition, create undesirable odours, and may soften the cured composition because of chain transfer reactions and, therefore, often requires the use of stabilizers.

Moreover, a redox curing dental composition comprising a radical polymerizable compound and a redox initiator system is required to have sufficient storage stability in order to be useful in practice. The formulation of the composition as a multi-pack composition for separating the reducing agent and the oxidizing agent of the redox initiator system during storage does not solve the inherent stability problem of the conventionally used components of a redox initiator system in a dental composition. On the other hand, an increase of the thermodynamic stability of the components of the initiator system usually leads to an increase of the kinetic stability and a reduced reactivity which may deteriorate working and setting times of the dental composition. Therefore, redox initiator systems are often caught in a stability/reactivity dilemma.

Finally, given that dental compositions usually contain (meth)acrylate or (meth)acrylamide monomers, free radical polymerization may be inhibited in the presence of oxygen forming stable peroxyl radicals with the growing polymer chains which are unable to or participate in a polymerization reaction. Oxygen inhibition may lead to premature chain termination and, therefore, incomplete curing.

A resin-modified dental cement is a particular dental composition comprising a reactive particulate glass and polyacidic polymer which is reactive with the reactive particulate filler in a cement reaction. Moreover, a resin-modified dental cement comprises radical polymerizable components. Accordingly, resin-modified dental cements are cured by two independent curing mechanisms including a cement reaction and a radical polymerization in an aqueous medium. When the resin-modified dental cement is formulated as an aqueous paste/paste system, the storage stability problem of the components may introduce severe limitations to the choice of a redox initiator system.

### Summary of the Invention

It is a problem of the present invention to provide an amine-free and peroxide-free redox curing dental composition, which may be efficiently cured in the absence of light, the composition having excellent properties with regard to storage stability and handling properties including suitable working time and a relatively short setting time of the uncured composition as well as having excellent physical and mechanical properties, biocompatibility, and aesthetics of the cured composition.

It is a further problem of the present invention to provide an amine-free and peroxide-free redox curing dental composition, wherein the oxygen inhibition can be controlled.

It is a further problem of the present invention to provide a redox initiator system which may be used in an amine-free and peroxide-free redox curing dental composition, which may be efficiently cured in the absence of light, the composition having excellent properties with regard to storage stability and handling properties including suitable working time and a relatively short setting time of the uncured composition as well as having excellent physical and mechanical properties, biocompatibility, and aesthetics of the cured composition.

Finally, it is a problem of the present invention the present invention to provide an amine-free and peroxide-free redox curing dental composition for use in the prevention and treatment of endodontic disease.

According to a first aspect, the present invention provides a redox curing dental composition comprising:
(a) one or more compounds having a radical polymerizable carbon-carbon double bond;
(b) a redox initiator system comprising
   (i) a reducing agent which is a compound of the following formula (I): 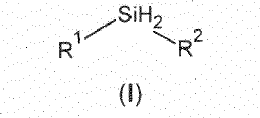 wherein
      R¹ and R²,
      which may be the same or different, independently represent a C₁₋₁₈ hydrocarbyl group, or R¹ and R² form together with the silicon atom to which they are bonded a 3 to 8-membered silicon-containing alicyclic ring, which groups R¹ and/or R² may be substituted with one to three substituents selected from the group consisting of a C₁₋₆ alkoxy group, a halogen atom, or a carboxylic acid group; and
   (ii) an oxidizing agent adapted to oxidize the reducing agent of formula (I) in the absence of light for generating a radical capable of initiating radical polymerization of the one or more compounds having a radical polymerizable carbon-carbon double bond.

In a specific embodiment, the present invention provides a redox curing dental composition according to the first aspect, wherein the redox initiator system further comprises a phosphine compound of the following formula (III): 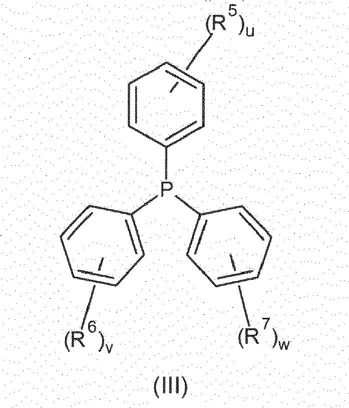 wherein
- R⁵: which may be the same or different when more than one R⁵ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group;
- R⁶: which may be the same or different when more than one R⁵ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group;
- R⁷: which may be the same or different when more than one R⁵ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group; or
two substituents selected from R⁵, R⁶ and R⁷ form together a single bond or a C₁₋₆ alkylene or C₂₋₆ alkenylene group; u, v and w, which may be the same or different, represent 0 or an integer of 1 to 5. According to a second aspect, the present invention provides a redox initiator system comprising
(i) a reducing agent which is a compound of the following formula (I): 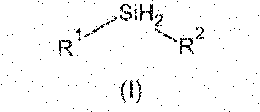 wherein
   R¹ and R²
   which may be the same or different, independently represent a C₁₋₁₈ hydrocarbyl group, or R¹ and R² form together with the silicon atom to which they are bonded a 3 to 8-membered silicon-containing alicyclic ring, which groups R¹ and/or R² may be substituted with one to three substituents selected from the group consisting of a C₁₋₆ alkoxy group, a halogen atom, or a carboxylic acid group; and
(ii) an oxidizing agent adapted to oxidize the reducing agent of formula (I) in the absence of light for generating a radical capable of initiating radical polymerization of the one or more compounds having a radical polymerizable carbon-carbon double bond.

According to a third aspect, the present invention provides the redox initiator system for use in the treatment or prevention of endodontic disease, in particular by root canal therapy.

A redox curing dental composition according to the present invention may be provided as a two-pack dental composition having high storage stability even when formulated as aqueous pastes, while at the same time providing high reactivity when the redox initiating system is activated by mixing the reducing agent and the oxidizing agent, and optionally by additional irradiation of light in the presence of a photosensitizer.

The present invention is based on the recognition that an amine-free and peroxide-free redox curing dental composition comprising one or more compounds having a radical polymerizable carbon-carbon double bond may be provided by using a redox initiator system containing a specific silane reducing agent and an oxidizing agent adapted to generate radicals by interaction with the reducing agent in the absence of light, whereby the redox curing dental composition may be efficiently cured in the absence of light. The redox curing dental composition of the present invention has excellent properties with regard to storage stability and handling properties including suitable working time and a relatively short setting time of the uncured composition as well as excellent physical and mechanical properties, biocompatibility, and aesthetics of the cured composition.

The redox curing dental composition of the present invention may further contain a specific phosphine compound whereby oxygen inhibition can be easily controlled without impairing the radical polymerization initiating efficiency of the redox initiator system.

A redox curing dental composition of the present invention may be formulated as a superior dental composition. Even when the redox curing dental composition of the present invention is a resin-modified dental cement formulated as an aqueous paste/paste composition, the composition has high storage stability and at the same time can be efficiently cured by a simultaneous cement and radical polymerization reaction.

When the specific silane reducing agent used in the redox initiator system of the present invention is combined with an oxidizing agent having an unsuitable reduction potential, in particular outside the range of from -0.15 to -1.2 V as determined by cyclic voltammetry, the silane reducing agent fails to generate any radicals which could initiate a polymerization reaction. However, when the silane reducing agent is oxidized with a mild oxidizing agent having a suitable reduction potential, the specific silane reducing agent used in the redox initiator system of the present invention efficiently generates radicals providing excellent curing rate and conversion.

A suitable oxidizing agent contains a metal cation or a cation of the following formula (II): 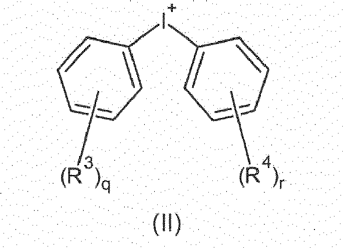 wherein
- R³: which may be the same or different when more than one R³ is present, independently represents a C₁₋₆ alkyl group, C₂₋₆ alkenyl group, a C₁₋₆ alkoxy group, a halogen atom, or a carboxylic acid group;
- R⁴: which may be the same or different when more than one R⁴ is present, independently represents, a C₁₋₆ alkyl group, C₂₋₆ alkenyl group, a C₁₋₆ alkoxy group, a halogen atom, or a carboxylic acid group; or
a substituent R³ and a substituent R⁴ form together a single bond or a C₁₋₆ alkylene or C₂₋₆ alkenylene group; and
q and r, which may be the same or different, represent 0 or an integer of 1 to 5

### Brief description of the Figures

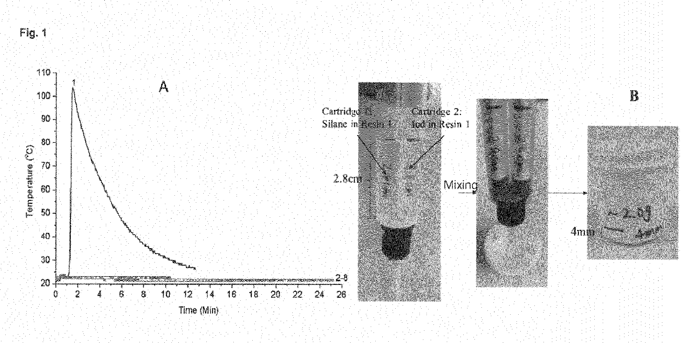

Fig. 1 shows the redox initiating profile for different two component silane and iodonium salt redox initiator systems.
   **A:** Optical pyrometric measurements (sample T (°C) vs time) under air for Resin 1 in presence of different RIS: (1-6) 1.0 wt % iodonium salt (in the following often referred to as lod) with 1.0 wt % (1) DPS; (2) DMPS; (3) MDPS; (4) TPS; (5) MTPS; (6) MODMPS; (7) DPS alone; (8) lod alone; 4 mm thick samples after mixing. **B:** Picture of mixing method of silane and lod for the optical pyrometric measurements (see also the experimental part).

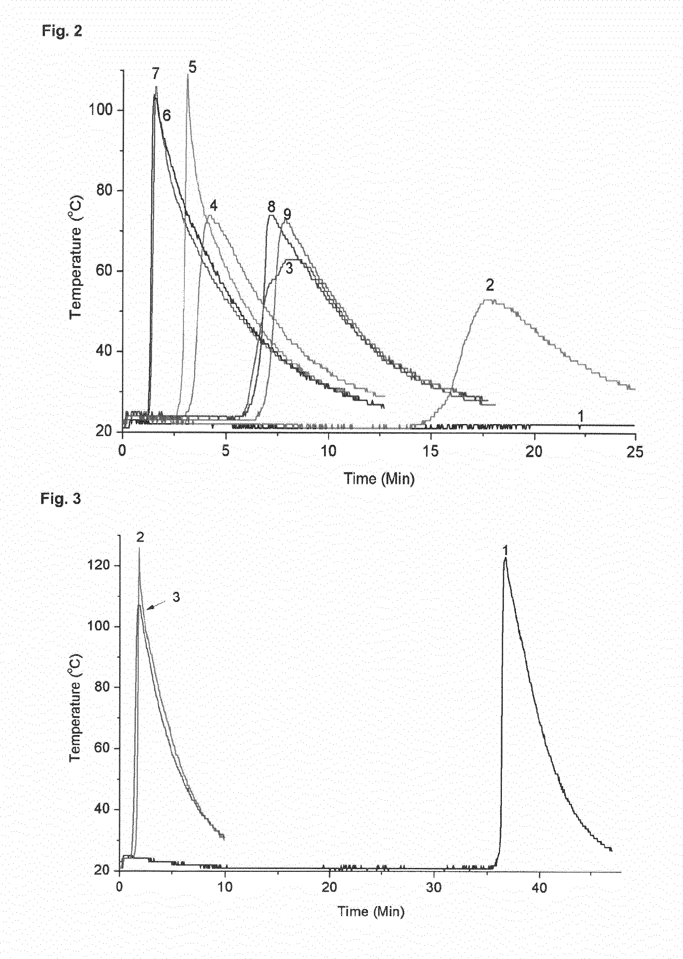

Fig. 2 shows the effect of the concentration of the redox initiator system on the initiating efficiency for Resin 1 at room temperature.
   Optical pyrometric measurements (sample T (°C) vs time under air of Resin 1 in presence of RIS, for: (1) 0.1 wt % DPS + 0.1 wt % lod; (2) 0.2 wt % DPS + 0.2 wt % lod; (3) 0.3 wt % DPS + 0.3 wt % lod; (4) 0.4 wt % DPS + 0.4 wt % lod; (5) 0.5 wt % DPS + 0.5 wt % lod; (6) 0.8 wt % DPS + 0.8 wt % lod; (7) 1.0 wt % DPS + 1.0 wt % lod; (8) 0.2 wt % DPS + 0.4 wt % lod; (9) 0.4 wt % DPS + 0.2 wt % lod; 4 mm thick samples.
Fig. 3 shows the initiating efficiency of the DPS/lod redox initiator system for different resins.
   Optical pyrometric measurements (sample T (°C) vs time) under air for different resins in presence of 0.5 wt % DPS + 0.5 wt % lod, for resin (1) Resin 4; (2) Resin1/Resin2 (50/50 w/w); (3) Resin1/Resin3 (50/50 w/w); 4 mm thick samples.
Fig. 4 shows the thermal initiating efficiency for the DPS/lod redox initiator system. Thermal polymerization experiments using DPS/lod as RIS (Enthalpy vs. heating temperature), heating rate: 10 K/min, under N₂, in Resin 1. For RIS: (1) 0.2 wt % DPS + 0.2 wt % lod; (2) 0.1 wt % DPS + 0.1 wt % lod; (3) 1.0 wt% DPS alone; (4) 1.0 wt% lod alone.
Fig. 5 shows the initiating efficiencies of different three-component redox initiator systems from DPS/lod/Phosphine having different phosphine additives.
   Optical pyrometric measurements (sample T (°C) vs time) under air of Resin 2 for **A:** (1) 1.0 wt% DPS+ 1.0 wt % lod + 1.0 wt % DMAPDP; (2) 0.5 wt% DPS+ 0.5 wt % lod + 0.5 wt % DMAPDP; (3) 2.0 wt % DPS + 2.0 wt % lod + 2.0 wt % 2DPPBA; (4) 1.0 wt % DPS + 1.0 wt % lod + 1.0 wt % 2DPPBA; (5) 2.0 wt % DPS + 2.0 wt % lod + 2.0 wt% 4DPPS; (6) 2.0 wt % DPS + 2.0 wt % lod; 4 mm thick samples. **B:** Picture of mixing method of DPS/lod and phosphine additives for the optical pyrometric measurements.

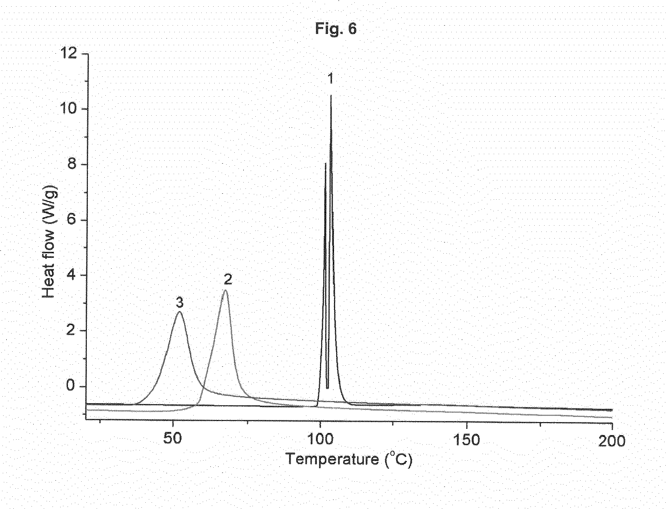

Fig. 6 shows thermal initiating efficiencies of DPS/lod/Phosphine redox initiator systems. Thermal polymerization experiments using DPS/lod/2DPPBA RIS (Enthalpy vs. heating temperature), heating rate: 10 K/min, under N₂, for Resin 2, For RIS: (1) 1.0 wt % DPS + 1.0 wt % lod; (2) 0.8 wt % DPS + 0.8 wt % lod + 0.8 wt% 2DPPBA; (3) 1.0 wt % DPS + 1.0 wt % lod + 1.0 wt% 2DPPBA.

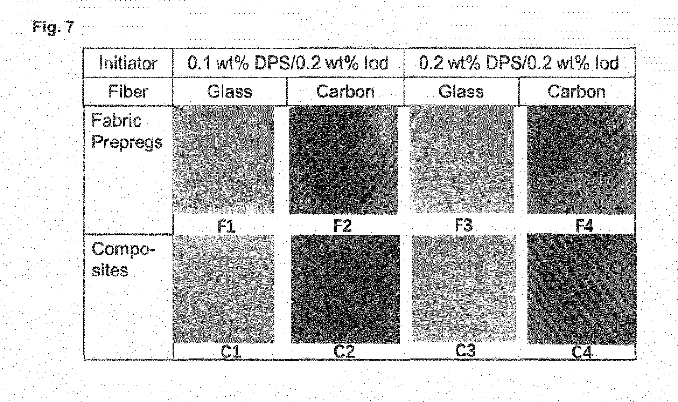

Fig. 7 shows results for the preparation of composites using the DPS/lod redox initiator system.
   Macrotopography of fabric prepregs (F1-F4) and composites (C1-C4) cured in presence of DPS/lod RIS at room temperature, under air. Access to prepregs **F1-F2** using: 0.1 wt% silane/0.2 wt% lod in: (F1) 50/50 (w/w) Resin 1/glass fibers; (F2) 50/50 (w/w) Resin 1/carbon fibers. Access to prepregs **F3-F4** using: 0.2 wt% DPS/0.2 wt% lod in: (F3) 50/50 (w/w) Resin 1/glass fibers; (F4) 50/50 (w/w) Resin 1/carbon fibers. Access to composites (**C1-C4**): at room temperature keeping for 10 min and heated 1 h at 100 °C under air.

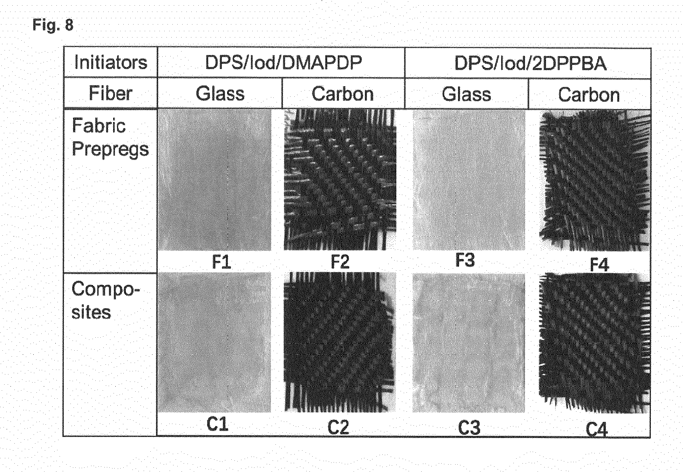

Fig. 8 shows results for the preparation of composites using the DPS/lod/phoshpine redox initiator system.
   Macrotopography of fabric prepregs (F1-F4) and composites (C1-C4) cured in presence of DPS/lod/phosphine RIS at room temperature, under air. Access to prepregs **F1-F2** using: 0.25 wt% DPS/0.25 wt% lod/0.25 wt% DMAPDP in: (F1) 50/50 (w/w) Resin 2/glass fibers; (F2) 50/50 (w/w) Resin 2/carbon fibers. Access to prepregs **F3 -F4** using: 0.5 wt% DPS/0.5 wt% lod/0.5 wt% 2DPPBA in: (F3) 50/50 (w/w) Resin 2/glass fibers; (F4) 50/50 (w/w) Resin 2/glass fibers. Access to composites (**C1-C4**): at room temperature keeping for (C1-C2) 10 min; (C3-C4) 90 min.

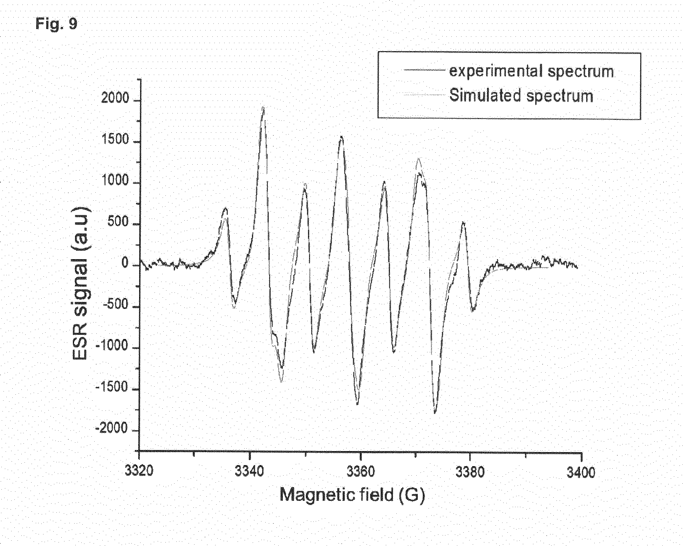

Fig. 9 shows an ESR spectra of an experiment using the DPS/lod redox initiator system. ESR spectra obtained for ESR-spin trapping experiment using [PBN] = 10⁻² M (as spin trap agent); [DPS] = 10⁻¹ M and [lod] = 10⁻² M in tert-butylbenzene; under N₂: simulated (red) and experimental (black) spectra.

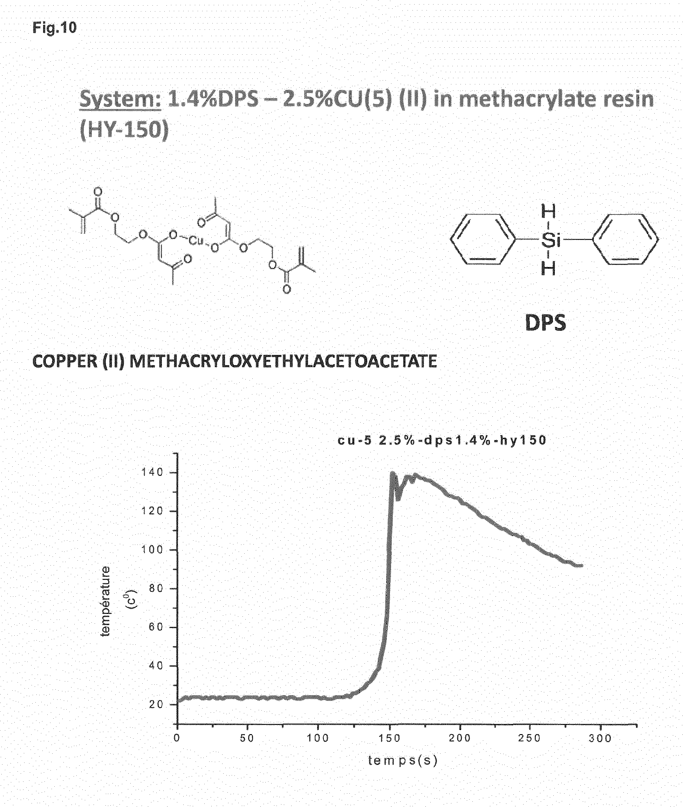

Fig. 10 shows the initiating efficiency for a redox initiator system using DPS and as an oxidizing agent using copper (II) methacryloxyethylacetoacetate.

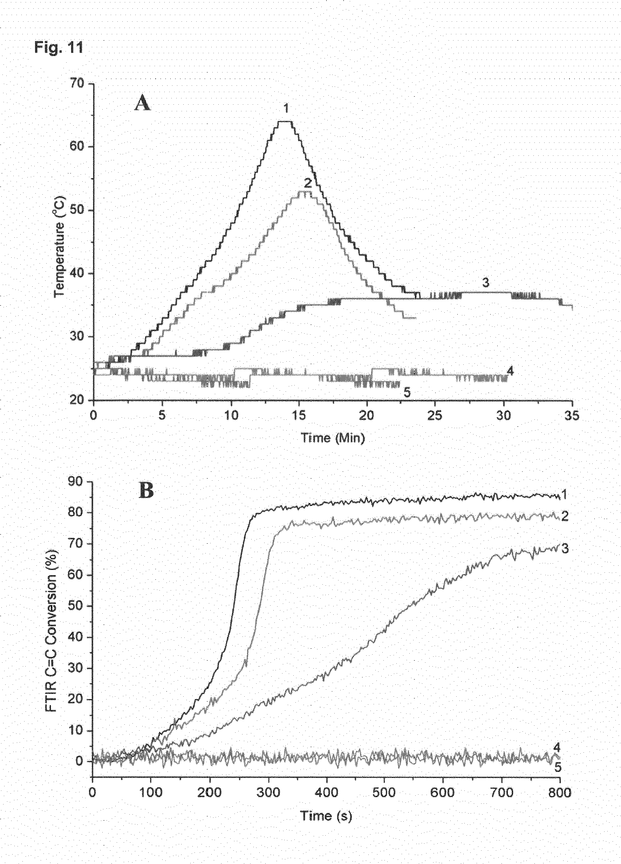

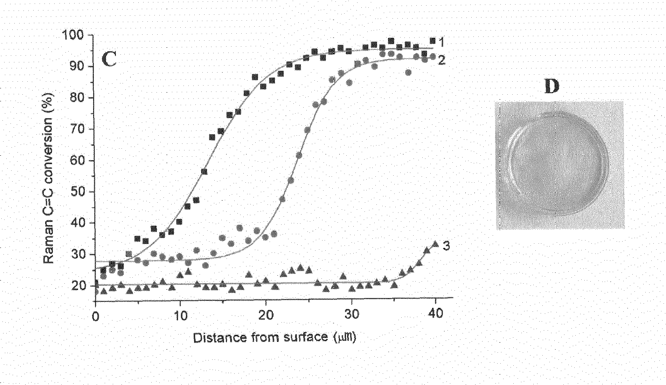

Fig. 11 shows the photo initiating efficiencies for the DPS/lod campherquinone three-component photo initiator system.
   **(A)** Optical pyrometric measurements (sample T (°C) vs. time) under air of Resin 2; 4 mm thick samples. **(B)** Photopolymerization profiles for different systems (C=C function conversion vs. irradiation time) measured by RT-FTIR for the photoinduced polymerization of Resin 2; 1.4 mm thick samples, under air. For the Initiating systems used in both **A** and **B:** (1) 1.0 wt% DPS + 1.0 wt% lod + 1.0 CQ under irradiation; (2) 1.0 wt% lod + 1.0 CQ under irradiation; (3) 1.0 wt% DPS + 1.0 CQ under irradiation; (4) 1.0 wt% DPS + 1.0 wt% lod under irradiation; (5) 1.0 wt% DPS + 1.0 wt% lod + 1.0 CQ without irradiation. For irradiation condition both **A** and **B:** upon LED@405nm irradiation (the irradiation starts at t=0 s, 110 mW/cm²). **C:** Raman conversion (C=C) as a function of the distance from the surface of the polymers (fitting curve 1, 2, 3 corresponding to the polymer obtained from optical pyrometric measurements of curve 1, 2, 3). **D:** Photo of the polymer obtained from optical pyrometric measurements.

### Detailed description of preferred embodiments

A hydrocarbyl group may be an alkyl group, a cycloalkyl group, a cycloalkylalkyl group, an arylalkyl group or an aryl group.

An alkyl group may be straight-chain or branched alkyl group, typically a C₁₋₆ alkyl group. Examples for a C₁₋₆ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl.

A cycloalkyl group may be a C₃₋₁₈ cycloalkyl group, typically a C₃₋₈ cycloalkyl group. Examples of the cycloalkyl group can include those having 3 to 6 carbon atoms, for example, cyclopropyl, cyclobutyl, cyclopentyl and cyclohexyl.

A cycloalkylalkyl group may have 4 to 18 carbon atoms and may include a combination of a linear or branched alkyl group having 1 to 6 carbon atoms and a cycloalkyl group having 3 to 14 carbon atoms. Examples of the cycloalkylalkyl(-) group can for example, include methylcyclopropyl(-) methylcyclobutyl(-), methylcyclopentyl(-), methylcyclohexyl(-), ethylcyclopropyl(-), ethylcyclobutyl(-), ethylcyclopentyl(-), ethylcyclohexyl(-), propylcyclopropyl(-), propylcyclobutyl(-), propylcyclopentyl(-), propylcyclohexyl(-).

An arylalkyl(-) group may be a C₇₋₁₈ arylalkyl(-) group, typically a combination of a linear or branched alkyl group having 1 to 6 carbon atoms and an aryl(-) group having 6 to 10 carbon atoms. Specific examples of an arylalkyl(-) group are a benzyl(-) group or a phenylethyl(-) group.

An aryl group can include aryl groups having 6 to 10 carbon atoms. Examples of the aryl group are phenyl and naphtyl.

An alkoxy group may be straight-chain or branched alkoxy group, typically a C₁₋₆ alkoxy group. Examples for a C₁₋₆ alkoxy group can include linear or branched alkoxy groups having 1 to 6 carbon atoms, preferably 1 to 4 carbon atoms, for example, methoxy, ethoxy, n-propoxy, isopropoxy, n-butoxy, isobutoxy, sec-butoxy, tert-butoxy, n-pentoxy, isopentoxy and n-hexoxy.

A halogen atom may be fluorine, chlorine, bromine or iodine, preferably fluorine.

The term "redox curing dental composition" refers to a dental composition which is self-curing by radical polymerization initiated by an initiator system activated in the absence of light. A redox curing dental composition may be further activated by irradiation of actinic light provided an additional photosensitizer is present in the composition. The additional photosensitizer has an absorption maximum in the range of from 400 to 800 nm.

The terms "polymerization" or "polymerizable" relate to the combination by covalent bonding of smaller molecules to form larger molecules, i.e. macromolecules or polymers. The monomers may be combined to form linear or crosslinked structures.

The term "polymerizable carbon-carbon double bond" as used herein in the context with component (a) means any double bond capable of addition polymerization, in particular free radical polymerization. Typically, compounds (a) include polymerizable carbon-carbon double bond selected from vinyl, conjugated vinyl, allyl, acryl, methacryl and styryl. More preferably, the polymerizable carbon-carbon double bond is selected from the group consisting of acryl, methacryl and styryl. Acryl and methacryl may be (meth)acryloyl or (meth)acrylamide. Most preferably, for compounds (a), the polymerizable double bound is acryl or methacryl.

The term "reactive particulate filler" refers to any particulate component capable of reacting with polyacidic polymer in a cement reaction. The term "cement reaction" means an acid-base reaction between the reactive particulate filler and the polyacidic polymer. Specifically, the reactive particulate filler is alkaline and reacts with the acid groups, such as carboxylic groups of the polyacidic polymer, whereby an acid-base reaction takes place resulting in the formation of ionic bonds.

The term "polyacidic" in connection with the polyacidic polymer means that the polymer has a plurality of acidic groups, preferably carboxylic acid groups, which may participate in a cement reaction with the reactive particulate filler. For example, the acidic groups in the form of carboxylic acid groups are preferably present in the backbone of the polymer and may be derived from (meth)acrylic acid, maleic acid and/or itaconic acid.

The term "initiator system" means any system of one or a mixture of two or more compounds that form free radicals when activated, e. g. by exposure to light and/or interaction with one or more further compounds in a photochemical or redox process, whereby polymerization of polymerizable compounds is initiated. An initiator system may be a photoinitiator system consisting of one or more initiator compounds generating alone or in combination free radicals when irradiated with light having a wavelength in the range of from 400 to 800 nm. Alternatively, the initiator system may be a redox initiator system consisting of two or more initiator compounds generating free radicals when mixed.

The term "initiator compound" in the context with the initiator system means any compound of the initiator system, for example a redox initiator component, a Norrish type I or II photoinitiator, an electron donor component, a sensitizer component or a coinitiator component. The term "photoinitiator" refers to any chemical compound that forms free radicals when activated, e. g. by exposure to light or interaction with a sensitizer in a photochemical process. The term "electron donor" refers to any compound which is capable of donating electrons in a photochemical process, for example organic compounds having heteroatoms with electron lone pairs, such as amine compounds. The term "sensitizer" refers to a molecule that produces a chemical change in another molecule such as the photoinitiator in a photochemical process. The term "photosensitizer" as used herein refers to any chemical compound that forms free radicals when activated, e. g. by exposure to light or interaction with a further compound, such as a coinitiator in a photochemical process.

The term "coinitiator" used in connection the photosensitizer refers to compounds which interact with the photosensitizer in the generation of radicals initiating a polymerization reaction, for example compounds having a tertiary amino group.

The term "curing" means the polymerization of functional polymerizable compounds such as monomers, oligomers or even polymers, into a polymer network, preferably a crosslinked polymer network.

The term "redox curing" refers to a dental composition that will radical polymerize into a polymer when the redox initiator contained in the dental composition is activated. The activation typically occurs by mixing an oxidizing agent and a reducing agent of the redox initiator.

The term "photocurable" refers to a dental composition that will radical and/or cationically polymerize into a polymer when irradiated for example with actinic radiation such as ultraviolet (UV), visible, or infrared radiation. "Actinic radiation" is any electromagnetic radiation that is capable of producing photochemical action and can have a wavelength of at least 150 nm and up to and including 1250 nm, and typically at least 300 nm and up to and including 750 nm.

The "working time" is the time between the beginning of the curing reaction, and the time the curing reaction proceeds to the point when it is no longer practical to perform further physical work upon the system, e.g. spatulate it or reshape it, for its intended dental or medical application.

The "setting time" is the time measured from the beginning of the curing reaction in a restoration to the time sufficient hardening has occurred to allow subsequent clinical or surgical procedures to be performed on the surface of the restoration. In a curing reaction, due to the presence of polymerizable carbon-carbon double bonds, a polymerization reaction takes place. In addition to the polymerization reaction, in a glass ionomer cement, the setting reaction additionally involves neutralization of acid groups, for example of polymerizable compounds, by a base in the form of a reactive particulate glass.

The term "endodontic disease" refers to damage to the dental pulp, commonly termed pulpitis, specifically irreversible pulpitis, which is a result of inflammation causing the tissues to swell and preventing blood from entering the root canal whereby the tooth "dies". The most common cause of irreversible pulpitis exposure of the pulp tissue to bacteria in the oral cavity whereby the inside of the tooth eventually fills with infected material that trickles through the openings in the tip of the root into the jaw. Since the bacteria have a secure hiding place inside the root canal, the immune system of the body is unable to clear up the infection, even with antibiotic treatment. One option to treat endodontic disease is root canal therapy.

### (a) Compounds having a radical polymerizable carbon-carbon double bond

A redox curing dental composition according to the present invention comprises one or more compounds having a radical polymerizable carbon-carbon double bond. In the following, the compound having a radical polymerizable carbon-carbon double bond may be referred to as "compound (a)". Suitable compounds (a) may be monomeric, oligomeric and polymeric.

The redox curing dental composition may comprise one or a mixture of two or more different compounds (a).

Each compound (a) may have one or more radical polymerizable carbon-carbon double bonds. The number of radical polymerizable carbon-carbon double bonds in a compound (a) is not particularly limited. In one embodiment of the present invention, the number of radical polymerizable carbon-carbon double bonds in a compound (a) is between 1 and 10, preferably between 1 and 5, more preferably between 1 and 4, still more preferably between 1 and 3, even more preferably 1 or 2. In a preferred embodiment the number of radical polymerizable carbon-carbon double bonds in a compound (a) is 1.

In one embodiment of the present invention, in a compound (a) the radical polymerizable carbon-carbon double bond may be selected from (meth)acryloyl group(s) and a (meth)acrylamide group(s). A (meth)acrylamide group may be an allyl (meth)acrylamide group.

Suitable examples for a compound (a) as a monomer may be selected from the group consisting of (meth)acrylates, amides of acrylic or methacrylic acid, urethane acrylates or methacrylates, and polyol acrylates or methacrylates.

(Meth)acrylates may be preferably selected from compounds of the following formulae (A), (B) and (C): 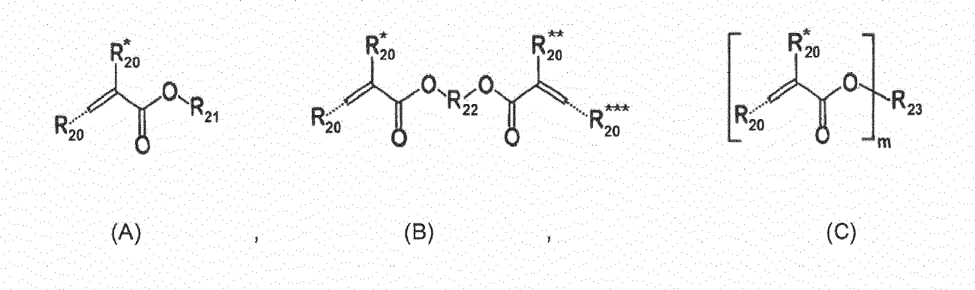
wherein R₂₀, R*₂₀, R**₂₀, R***₂₀ independently represent a hydrogen atom, -COOM^{y}, a linear C₁₋₁₈ or branched C₃₋₁₈ alkyl group which may be substituted by a C₃₋₆ cycloalkyl group, a C₆₋₁₄ aryl or C₃₋₁₄ heteroaryl group, -COOM^{y}, -PO₃M^{y}, -O-PO₃M^{y}₂ or -SO₃M*, a C₃ to C₁₈ cycloalkyl group which may be substituted by a C₁₋₁₆ alkyl group, a C₆₋₁₄ aryl or C₃₋₁₄ heteroaryl group, or a C₅ to C₁₈ aryl or C₃ to C₁₈ heteroaryl group, -COOM^{y}, -PO₃M^{y}, -O-PO₃M^{y}₂ or -SO₃M*,
R₂₁ represents a hydrogen atom, a linear C₁₋₁₈ or branched C₃₋₁₈ alkyl group or C₂ to C₁₈ alkenyl group which may be substituted by a C₃₋₆ cycloalkyl group, a C₆₋₁₄ aryl or C₃₋₁₄ heteroaryl group, -COOM^{y}, -PO₃M^{y}, -O-PO₃M^{y}₂ or -SO₃M*, a C₃ to C₁₈ cycloalkyl group which may be substituted by a C₁₋₁₆ alkyl group, a C₆₋₁₄ aryl or C₃₋₁₄ heteroaryl group,-COOM^{y}, -PO₃M^{y}, -O-PO₃M^{y}₂ or -SO₃M*, or a C₅ to C₁₈ aryl or C₃ to C₁₈ heteroaryl group, R₂₂ represents a divalent organic residue having from 1 to 45 carbon atoms, whereby the divalent organic residue may contain at least one of from 1 to 7 C₃₋₁₂ cycloalkylene group(s), 1 to 7 C₆₋₁₄ arylene groups, 1 to 7 carbonyl groups, 1 to 7 carboxyl groups (-(C=O)-O- or -O-(C=O-), 1 to 7 amide groups (-(C=O)-NH- or -NH-(C=O)-) or 1 to 7 urethane groups (-NH-(C=O)-O- or -O-(C=O)-NH-), and 1 to 14 heteroatoms selected from oxygen, nitrogen and sulphur, which divalent organic residue may be substituted with one or more substituents selected from the group consting of a hydroxyl group, a thiol group, a C₆₋₁₄ aryl group,-COOM^{y}, -PO₃M^{y}, -O-PO₃M^{y}₂ or -SO₃M*; preferably R₂₂ is a C₁ to C₁₈ alkylene group or a C₂ to C₁₈ alkenylene group, which may be substituted by one or more -OH group(s), which alkylene or alkenylene group may contain at least one of 1 to 4 C₆₋₁₀ arylene groups, 1 to 4 urethane groups (-NH-(C=O)-O- or -O-(C=O)-NH-), and 1 to 8 oxygen atoms;
R₂₃ represents a saturated di- or multivalent substituted or unsubstituted C₂ to C₁₈ hydrocarbon group, a saturated di- or multivalent substituted or unsubstituted cyclic C₃ to C₁₈ hydrocarbon group, a di- or multivalent substituted or unsubstituted C₄ to C₁₈ aryl or heteroaryl group, a di- or multivalent substituted or unsubstituted C₅ to C₁₈ alkylaryl or alkylheteroaryl group, a di- or multivalent substituted or unsubstituted C₇ to C₃₀ aralkyl group, or a di- or multivalent substituted or unsubstituted C₂ to C₄₅ mono-, di-, or polyether residue having from 1 to 14 oxygen atoms, and
m is an integer, preferably in the range from 1 to 10,
wherein M^{y} of any one of R₂₀, R*₂₀, R**₂₀, R***₂₀, R₂₁, and R₂₂, which M^{y} are independent from each other, each represent a hydrogen atom or a metal atom, and
M* of any one of R₂₀, R*₂₀, R**₂₀, R***₂₀, R₂₁, and R₂₂, which M* are independent from each other, each represent a metal atom.

For R₂₀, R*₂₀, R**₂₀ and R***₂₀, the linear C₁₋₁₈ or branched C₃₋₁₈ alkyl group may e.g. be methyl, ethyl, n-propyl, i-propyl, n-butyl, isobutyl, tert-butyl, sec-butyl, pentyl or hexyl. For R₂₁ and R*₂₁, the C₁₋₁₈ alkyl group or C₂₋₁₈ alkenyl group may e.g. be eth(en)yl, n-prop(en)yl, i-prop(en)yl , n-but(en)yl, isobut(en)yl, tert-but(en)yl sec-but(en)yl, pent(en)yl or hex(en)yl.

For R₂₀, R*₂₀, R**₂₀, R***₂₀ and R₂₁ an aryl group may, for example, be a phenyl group or a naphthyl group, and a C₃₋₁₄ heteroaryl group may contain 1 to 3 heteroatoms selected from nitrogen, oxygen and sulfur.

For R₂₂, in the phrase "divalent organic residue may contain at least one of ..." means that the groups which may be contained in the divalent organic residue are incorporated in the divalent organic residue by means of covalent bonding. For example, in BisGMA, two aryl groups in the form of phenyl and two heteroatoms in the form of oxygen are incorporated into the divalent organic residue of R₂₂. Or, as a further example, in UDMA, two urethane groups (-NH-(C=O)-O- or -O-(C=O)-NH-) are incorporated in the divalent organic residue of R₂₂.

In formula (B), the dotted bond indicates that R₂₀ and R***₂₀ may be in *(Z)* or *(E)* configuration relative to CO.

Preferably, in formulae (A), (B) and (C), R₂₀, R*₂₀, R**₂₀ and R***₂₀ independently represent a hydrogen atom, a linear C₁₋₁₆ or branched C₃₋₁₆ alkyl group which may be substituted by a C₃₋₆ cycloalkyl group, a C₆₋₁₄ aryl or C₃₋₁₄ heteroaryl group, a C₃₋₆ cycloalkyl group which may be substituted by a C₁₋₁₆ alkyl group, a C₆₋₁₄ aryl or C₃₋₁₄ heteroaryl group, a C₆₋₁₄ aryl or C₃₋₁₄ heteroaryl group. More preferably, in formula (B), R₂₀, R*₂₀, R**₂₀ and R***₂₀ independently represent a hydrogen atom, a linear C₁₋₈ or branched C₃₋₈ alkyl group which may be substituted by a C₄₋₆ cycloalkyl group, a C₆₋₁₀ aryl or C₄₋₁₀ heteroaryl group, a C₄₋₆ cycloalkyl group which may be substituted by a C₁₋₆ alkyl group, a C₆₋₁₀ aryl or C₄₋₁₀ heteroaryl group or a C₆₋₁₀ aryl group. Even more preferably, R₂₀, R*₂₀, R**₂₀ and R***₂₀ independently represent a hydrogen atom, a linear C₁₋₄ or branched C₃ or C₄ alkyl group which may be substituted by a cyclohexyl group or a phenyl group, or a cyclohexyl group which may be substituted by a C₁₋₄ alkyl group. Most preferably, R₂₀, R*₂₀, R**₂₀ and R***₂₀ independently represent a hydrogen atom or a linear C₁₋₄ or branched C₃ or C₄ alkyl group.

Preferably, in formula (A), R₂₁ represents a hydrogen atom, a linear C₁₋₁₆ or branched C₃₋₁₆ alkyl group or C₂₋₁₆ alkenyl group which may be substituted by a C₃₋₆ cycloalkyl group, a C₆₋₁₄ aryl or C₃₋₁₄ heteroaryl group, a C₃₋₆ cycloalkyl group which may be substituted by a C₁₋₁₆ alkyl group, a C₆₋₁₄ aryl or C₃₋₁₄ heteroaryl group, a C₆₋₁₄ aryl or C₃₋₁₄ heteroaryl group. More preferably, R₂₁ represents a hydrogen atom, a linear C₁₋₁₀ or branched C₃₋₁₀ alkyl or C₂₋₁₀ alkenyl group group which may be substituted by a C₄₋₆ cycloalkyl group, a C₆₋₁₀ aryl or C₄₋₁₀ heteroaryl group, a C₄₋₆ cycloalkyl group which may be substituted by a C₁₋₆ alkyl group, a C₆₋₁₀ aryl or C₄₋₁₀ heteroaryl group or a C₆₋₁₀ aryl group. Even more preferably, R₂₁ represents is a hydrogen atom, a linear C₁₋₁₀ or branched C₃₋₁₀ alkyl group or linear C₂₋₁₀ or branched C₃₋₁₀ alkenyl group which may be substituted by a cyclohexyl group or a phenyl group, or a cyclohexyl group which may be substituted by a C₁₋₄ alkyl group. Yet even more preferably, R₂₁ represents an unsubstituted C₁₋₁₀ alkyl group or C₂₋₁₀ alkenyl group, still even more preferably an unsubstituted C₂₋₆ alkyl group or C₃₋₆ alkenyl group, and most preferably an ethyl group or an allyl group.

The (meth)acrylate compounds of formulae (A), (B) and (C) may be selected from the group consisting of methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, bisphenol A glycerolate dimethacrylat ("bis-GMA", CAS-No. 1565-94-2), 4,4,6,16 (or 4,6,6,16)-tetramethyl-10,15-dioxo-11,14-dioxa-2,9-diazaheptadec-16-enoicacid 2-[(2-methyl-1-oxo-2-propen-1-yl)oxy]ethyl ester (CAS no. 72869-86-4)_(UDMA), glycerol mono-and di- acrylate such as 1,3-glycerol dimethacrylate (GDM), glycerol mono-and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acrylate]propane.

Most preferably, a compound of formula (B) is selected from the group consisting of:

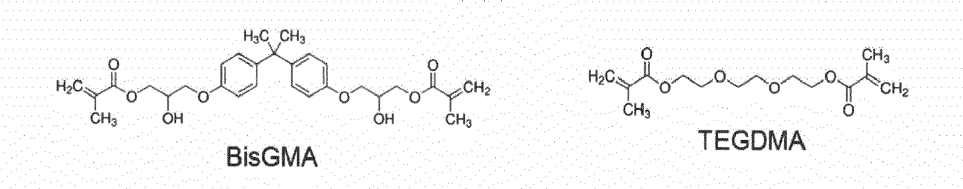

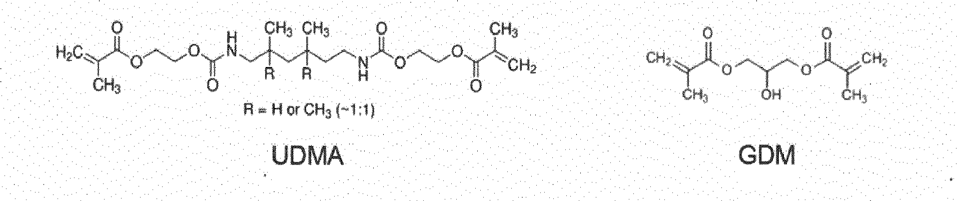

Particular preferred mono- or bis- or (meth)acrylamides and poly[(meth) acrylamides] have the following formulae (D), (E) and (F): 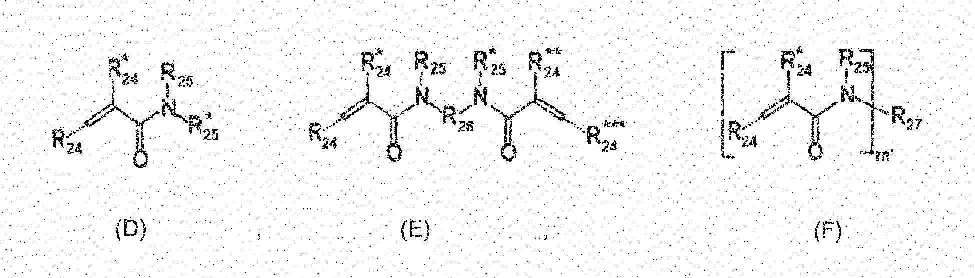 wherein R₂₄ R*₂₄, R**₂₄, R***₂₄ have the same meaning as R₂₀ R*₂₀, R**₂₀, R***₂₀ defined above for formulae (A), (B) and (C), R₂₅, R*₂₅ independently represent a residue having the same meaning as R₂₁ defined above for formula (A), and R₂₇ and m' have the same meaning as R₂₃ and m defined above for formula (C).

In formula (E), R₂₆ represents a divalent substituted or unsubstituted organic residue having from 1 to 45 carbon atoms, whereby said organic residue may contain at least one of 1 to 7 C₃₋₁₂ cycloalkylene group(s), 1 to 7 C₆₋₁₄ arylene groups, from 1 to 7 carbonyl groups, 1 to 7 carboxyl groups (-(C=O)-O- or -O-(C=O-), 1 to 7 amide groups (-(C=O)-NH- or -NH-(C=O)-), 1 to 7 urethane groups (-NH-(C=O)-O- or -O-(C=O)-NH-), and 1 to 14 heteroatoms selected from oxygen, nitrogen and sulphur, which divalent organic residue may be substituted with one or more substituent(s) selected from the group consisting of a hydroxyl group, a thiol group, a C₆₋₁₄ aryl group, -COOM, -PO₃M, -O-PO₃M₂ or -SO₃M* Preferably, R₂₆ is a C₁ to C₁₈ alkylene group or a C₂ to C₁₈ alkenylene group which may contain at least one of 1 to 4 C₆₋₁₀ arylene groups and C₃₋₈ cycloalkylene group, 1 to 4 urethane groups (-NH-(C=O)-O- or -O-(C=O)-NH-), and 1 to 8 oxygen atoms or nitrogen atoms.

For R₂₆, the phrase "divalent organic residue may contain at least one of ..." has an analogous meaning as defined above for R₂₂ of compound of formula (B).

In formulae (D), (E), (F), the dotted bond indicates that R₂₄ and R***₂₄ may be in *(Z)* or *(E)* configuration relative to CO.

In compound of formula (D), R₂₅ and R₂₅* may cooperatively form a ring in which R₂₅ and R₂₅* are linked by a C-C bond or a functional group selected from the group consisting of an ether group, a thioether group, an amine group and an amide group.

Preferred methacrylamides according to formulae (D), (E), (F) have the following formulae:

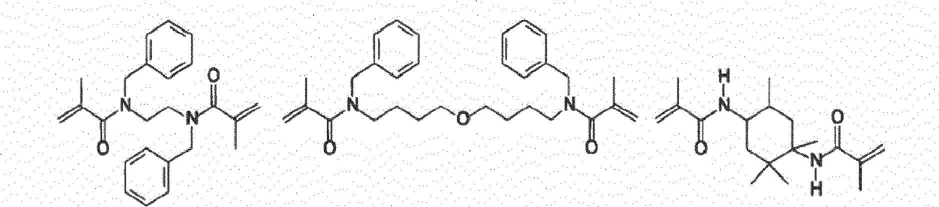

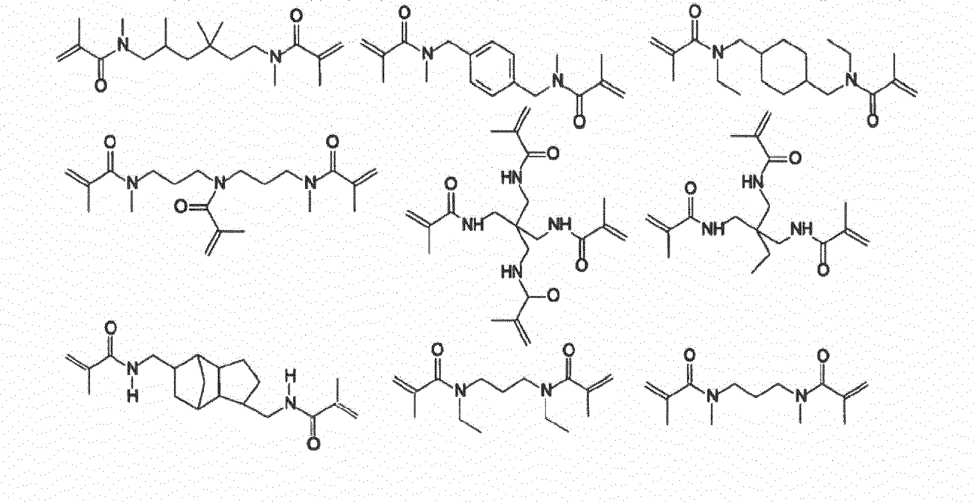

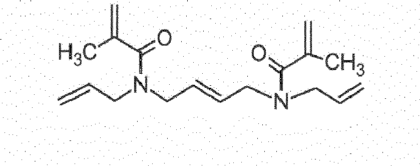

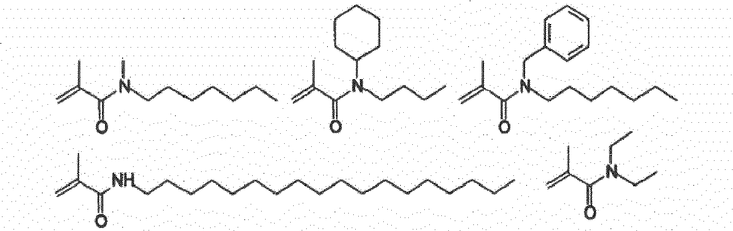

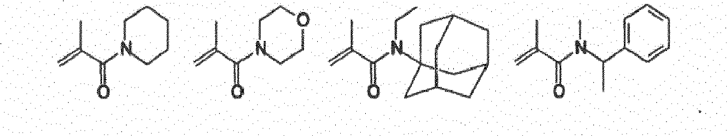

Preferred acrylamides according to formulae (D), (E), (F) have the following formulae:

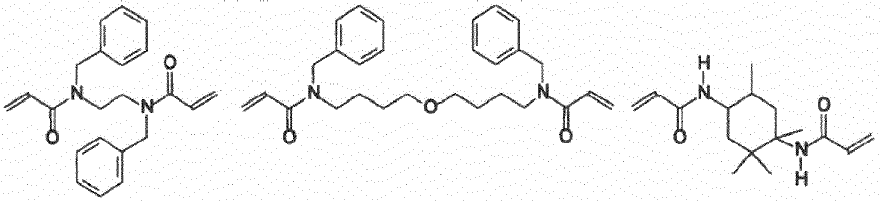

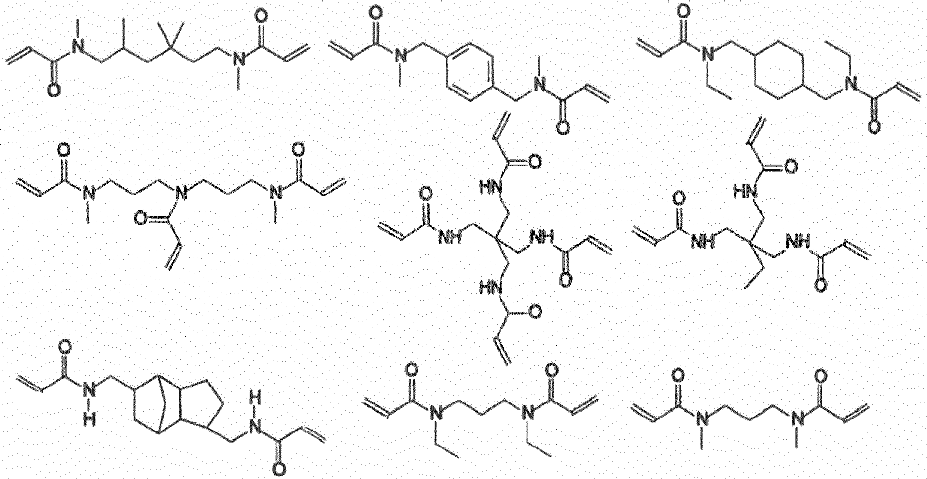

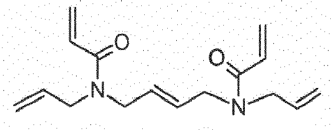

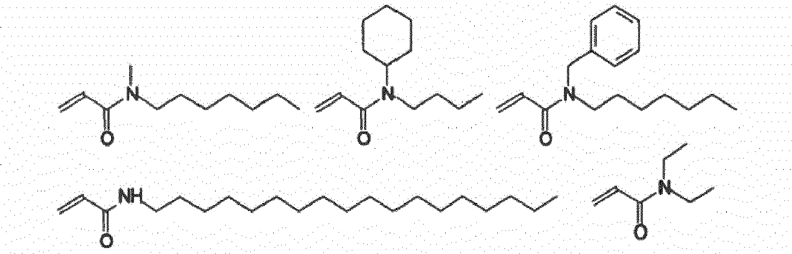

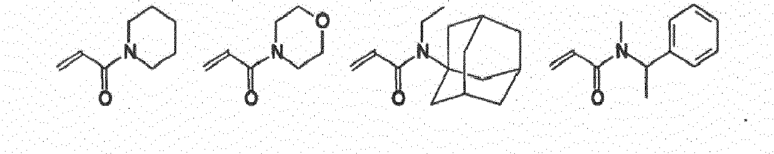

Most preferred are the bis-(meth)acrylamides:
N,N'-diallyl-1,4-bisacrylamido-(2E)-but-2-en (BAABE) having the structural formula 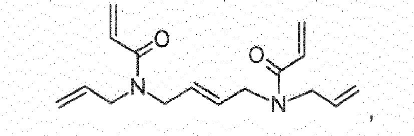 and
N,N'-diethyl-1,3-bisacrylamido-propan (BADEP) having the structural formula

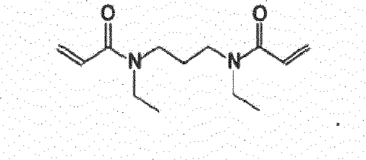

A compound (a) having a (meth)acryloyl group or a (meth)acrylamide group may also be selected from phosphoric acid ester group containing polymerizable monomers having at least one radical polymerizable carbon-carbon double bond. Preferably, such phosphoric acid ester group containing polymerizable monomers have the following formula (G): 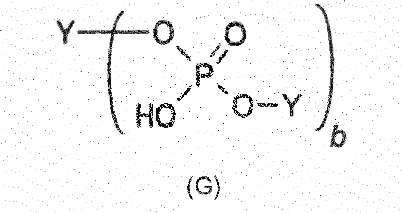 wherein
the moieties Y independent from each other represent a hydrogen atom or a moiety of the following formulae (Y*), (Y**) or (Y***): 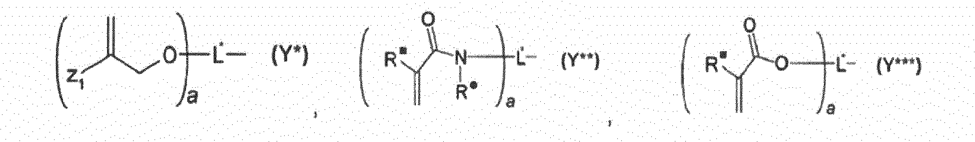 wherein
Z₁ is COOR^{α}, COSR^{β}, CON(R^{α})₂, CONR^{α}R^{β}, or CONHR^{α}, wherein R^{α} and R^{β} independently represent a hydrogen atom, a C₁₋₁₈ alkyl group optionally substituted by a C₃₋₈ cycloalkyl group, an optionally substituted C₃₋₈ cycloalkyl group, an optionally substituted C₄₋₁₈ aryl or heteroaryl group, an optionally substituted C₅₋₁₈ alkylaryl or alkylheteroaryl group, or an optionally substituted C₇₋₃₀ aralkyl group, whereby two R¹³ residues may form together with the adjacent nitrogen atom to which they are bound a 5- to 7-membered heterocyclic ring which may contain further nitrogen atoms or an oxygen atoms, and whereby the optionally substituted groups may be substituted by 1 to 5 C₁₋₅ alkyl group(s);
R^{■} and R^{•} independently represent a hydrogen atom, an optionally substituted C₁₋₁₈ alkyl group, an optionally substituted C₃₋₁₈ cycloalkyl group, an optionally substituted C₅₋₁₈ aryl or heteroaryl group, an optionally substituted C₅₋₁₈ alkylaryl or alkylheteroaryl group, an optionally substituted C₇₋₃₀ aralkyl group, whereby the optionally substituted groups may be substituted by 1 to 5 C₁₋₅ alkyl group(s);
L* represents an (*a*+*b*)-valent organic residue (whereby b is 1 when Y in formula (D) is within the round brackets) containing 2 to 45 carbon atoms and optionally heteroatoms such as oxygen, nitrogen and sulfur atoms, the carbon atoms including a + b carbon atoms selected from primary and secondary aliphatic carbon atoms, secondary alicyclic carbon atoms, and aromatic carbon atoms, each of the a+b carbon atoms linking a phosphate or a moiety of any one of formula (Y*), (Y**) and (Y***); a is an integer of from 1 to 10, preferably 1 to 5; *b* is an integer of from 1 to 10, preferably 1 to 5; provided that at least one Y is not hydrogen. The preparation of such compounds wherein Y = Y* is known from EP 1 548 021 A1.

Furthermore, a compound (a) having a (meth)acryloyl group or a (meth)acrylamide group may also be selected from phosphonic acid group containing polymerizable acidic compounds of the following formula (H): 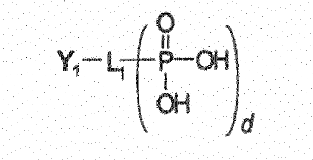 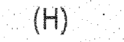 wherein
the moiety Y₁ represents a moiety of the following formulae (Y₁**) or (Y₁***): 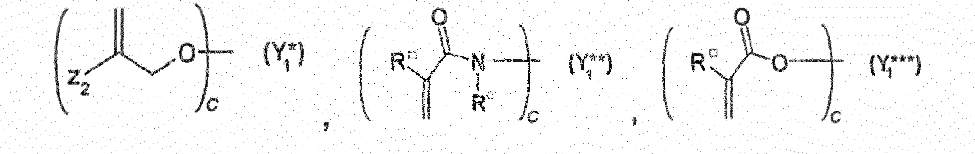
Z₂ independently has the same meaning as defined for Z₁;
R^{□} and R^{o} independently have the same meaning as defined for R^{■} and R^{•};
L₁ represents a (*c* + *d*) valent organic residue containing 2 to 45 carbon atoms and optionally heteroatoms such as oxygen, nitrogen and sulfur, the carbon atoms including c + d carbon atoms selected from primary and secondary aliphatic carbon atoms, secondary alicyclic carbon atoms, and aromatic carbon atoms, each of the c+d carbon atoms linking a phosphonate or a moiety of any one of formula (Y₁*), (Y₁**) and (Y₁***); and c and d independently represent integers of from 1 to 10.

From compound of formula (G'), the following formulae are particularly preferred: 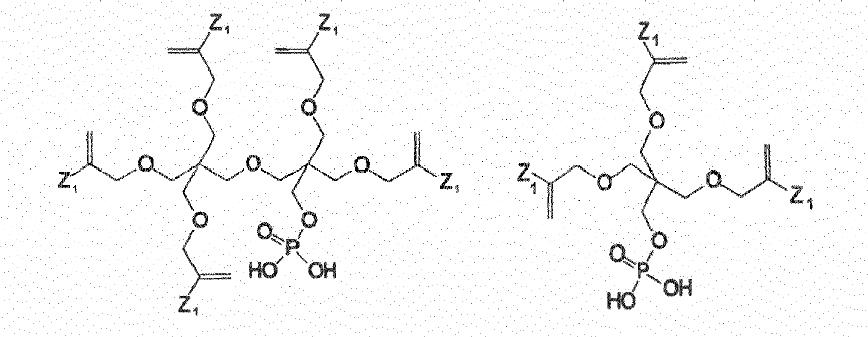 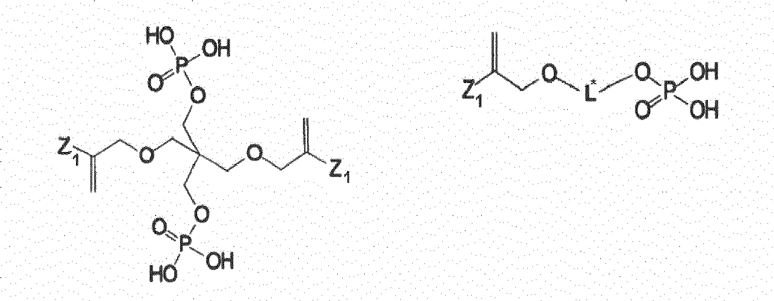 , wherein Z₁ is defined as above, and L* is an optionally substituted alkylene group. More preferably, Z₁ is methyl, and L* is a C₄ to C₁₆ alkylene group. Even more preferably, L* is a C₈ to C₁₂ alkylene group.

Furthermore, a compound (a) having one or more radical polymerizable carbon-carbon double bond(s) may be selected from the hydrolysis stable polyfunctional polymerizable monomers disclosed in EP 2 705 827 and EP 2 727 576.

Particularly preferred compound(s) (a) are selected from the compounds of formulae (D), (E), (F), (G) and (H), more preferably from the compounds of formulae (D), (E), (F), and most preferably from compounds of formula (E).

Compound(s) (a) in the form of polymers are preferably selected from polymerizable polyacidic polymers. The term "polymerizable" as used with the term "polymerizable polyacidic polymer" means a polymer having a radical polymerizable carbon-carbon double bond. The "polymerizable polyacidic polymer" may be combined with a crosslinker as well as e.g. with a monomer having polymerizable carbon-carbon double bond, to form graft polymers and/or crosslinked polymers when curing the dental composition.

The term "polyacidic" as used with the term "polymerizable polyacidic polymer" means that the polymer has a plurality of acidic groups, preferably carboxylic acid groups, which may participate in a cement reaction with a reactive glass. The carboxylic acid groups are preferably present in the backbone and derived from acrylic acid, methacrylic acid and/or itaconic acid. Additional acidity may be introduced by carboxylic acid groups in the group of formula (V) and carboxylic group(s) in the optional repeating unit of formula (VI).

A particularly preferred radical polymerizable polyacidic polymer has repeating units of the following formula (IV): 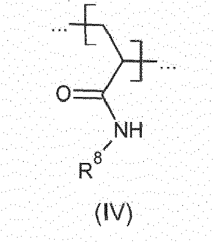 wherein R⁸ represents a group of the following formula (V):

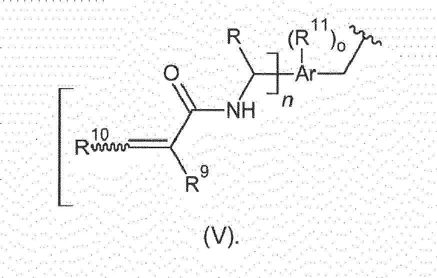

In formulae (V) and (VI), the jagged bond indicates that R¹⁰ may be in *cis* or *trans* configuration relative to the carbonyl group. Furthermore, in formula (V), the jagged line which is perpendicular to a normal bond line indicates the attachment of R⁸ to the nitrogen of the amide moiety of the repeating unit of formula (IV). In formula (VIII), the jagged line perpendicular to a normal bond indicates the attachment of R¹³ to the nitrogen of the amide moiety of the repeating unit of formula (VII).

The radical polymerizable polyacidic polymer having repeating units of formula (IV) is water-soluble and is reactive with a particulate glass in a cement reaction, whereby the radical polymerizable polyacidic polymer has a polymer backbone and hydrolysis-stable pendant groups R⁸ having one or more radical polymerizable carbon-carbon double bonds.

The polymerizable pendant groups R⁸ of the radical polymerizable polyacidic polymer having repeating units of formula (IV) may react with a monomer having a radical polymerizable double bond, whereby a graft polymer is formed. The grafted side-chains may contain additional carboxylic acid groups which can take part in a cement reaction, thereby further increasing the strength of the cured composition.

In formula (V), Ar is an aromatic group which may be further substituted. The aromatic group is not specifically limited and may be any organic aromatic group, i.e. a cyclic moiety which number of π-electrons equals 4*n*+2, where *n* is zero or any positive integer. Preferably, Ar is derived from an arene or heteroarene. An arene is a monoyclic or polycyclic aromatic hydrocarbon. A heteroarene is a heterocyclic compound formally derived from arenes by replacement of one or more methine (-C=) and/or vinylene (-CH=CH-) groups by trivalent or divalent heteroatoms, respectively, in such a way as to maintain the continuous π-electron system characteristic of aromatic systems and a number of out-of-plane π-electrons corresponding to the Hückel rule (4 n + 2).

In case o+n is 2, Ar is preferably a C₆₋₁₄ arenetriyl or C₃₋₁₄ heteroarenetriyl group which may be further substituted by one or more substituents. In case o + n is 3, Ar is preferably a C₆₋₁₄ arenetetrayl or C₃₋₁₄ heteroarenetetrayl group which may be further substituted by one or more additional substituents. In case o + n is 4, Ar is preferably a C₆₋₁₄ arenepentayl or C₃₋₁₄ heteroarenepentayl group which may be further substituted by one or more additional substituents. In case o + n is 5, then Ar is preferably a C₆₋₁₄ arenehexayl or C₃₋₁₄ heteroarenehexayl group which may be further substituted by one or more additional substituent.

The additional substituents are selected from the group consisting of a straight chain or branched C₁ to C₁₀ alkyl group, a straight chain or branched C₁ to C₁₀ alkenyl group,-COOM^{x}, -PO₃M^{x}, -O-PO₃M^{x}₂ and -SO₃M^{x}, wherein M^{x} represents a hydrogen atom or a metal atom. More preferably, Ar is a C₆₋₁₀ arenetriyl or C₃₋₉ heteroarenetriyl group which may be substituted by one or more additional substituents selected from a straight chain or branched C₁ to C₄ alkyl group and a straight chain or branched C₁ to C₄ alkenyl group. Even more preferably, Ar is selected from a benzenetriyl group, a naphtalenetriyl group, a toluenetriyl group, a xylenetriyl group and a styrenetriyl group, and the heteroaryl group is a pyridinetriyl group. Yet even more preferably, Ar is a benzenetriyl group. Most preferably, Ar is a benzenetriyl group wherein a hydroxyl group is present in formula (V) in para-position to the methylene group linking R⁸.

In formula (V), R⁹ and R¹⁰, which may be the same or different, independently represent a hydrogen atom, or a C₁₋₆ alkyl group which may be substituted with a carboxylic acid group. Preferably, R⁹ and R¹⁰, which may be the same or different, independently represent a hydrogen atom or a C₁₋₃ alkyl group. More preferably, R⁹ represents a hydrogen atom or a methyl group, and R¹⁰ represents a hydrogen atom. Most preferably, both R⁹ and R¹⁰ represent a hydrogen atom.

In formula (V), one or more R may be present depending on the value of n. The R may be the same or different when more than one R is present. R represents a hydrogen atom, a carboxylic acid group, a COOR^{a} group, a CONHR^{b} group, or a CONR^{c}₂ group. R^{a}, R^{b}, and R^{c} represent a C₁₋₆ alkyl group. According to a preferred embodiment, R represents a hydrogen atom.

In formula (V), R¹¹ represents an electron donating group which activates the aryl group. Accordingly, each R¹¹ is directly bonded to a ring atom of the Ar group. R¹¹ may be a halogen atom or a group selected from -OH, -OR^{d}, -NR^{e}H, -NR^{e}R^{f}, -SH, and -SR^{g}, wherein R^{d}, R^{e}, R^{f}, R^{g}, and R^{g}, represent a C₁₋₆ alkyl group. Preferably, R¹¹ is a hydroxyl group. The halogen atom may be a fluorine atom, a chlorine atom, a bromine atom or an iodine atom. When o is 2, the R¹¹ cannot be both OH.

In formula (V), o is an integer of 1 or 2. Preferably, o is 1. In formula (V), n is an integer of 1 to 4. Preferably, n is an integer of 1 or 2. In formula (V), o+n is preferably 5 or less, more preferably 4 or less, in particular 3.

It is preferred that in formula (V), Ar is a phenyl group. Specifically, R⁸ preferably represents a group of the following formula (V'): 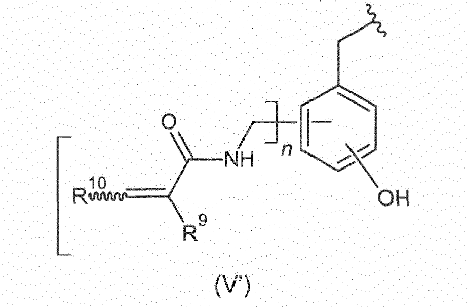 wherein R⁹, R¹⁰ and n are as defined as above.

It is particularly preferred that R⁸ is a group of the following formula (V"ₐ) or (V"_{b}):

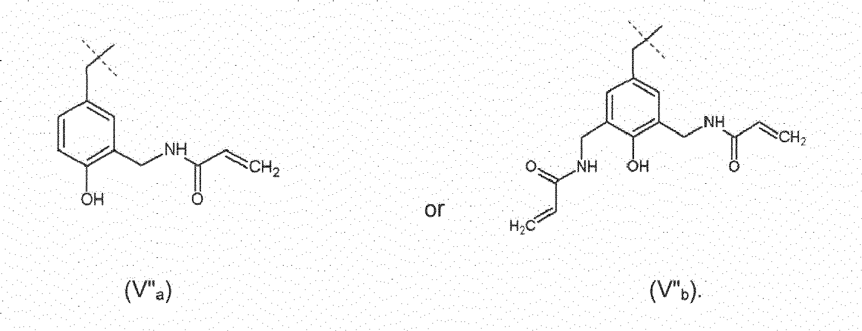

Furthermore, it is preferred that the radical polymerizable polyacidic polymer having repeating units of formula (IV) further comprises acidic repeating units of the following formula (VI):

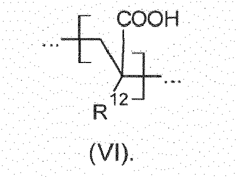

In formula (VI), R¹² represents a hydrogen atom, or a C₁₋₆ alkyl group which may be substituted with a carboxylic acid group. Preferably, R¹² represents a hydrogen atom, or a C₁₋₃ alkyl group which may be substituted with a carboxylic acid group, more preferably R¹² represents a hydrogen atom or a methyl group. Most preferably, R¹² represents a hydrogen atom.

In the radical polymerizable polyacidic polymer having repeating units of formula (IV), the molar ratio of repeating units of formula (VI) and repeating units of formula (IV) ([formula (VI)]/[formula (IV)]) is preferably in the range of 1000:1 to 1:1, more preferably 100:1 to 5:1, most preferably 50:1 to 10:1.

The radical polymerizable polyacidic polymer having repeating units of formula (IV) preferably has a molecular weight M_{w} in the range of 10,000 to 250,000, more preferably 20,000 to 150,000, most preferably 30,000 to 100,000.

The radical polymerizable polyacidic polymer having repeating units of formula (IV) is hydrolysis stable, which means that it does not contain groups hydrolysing at pH 2.5 within one month when stored at a temperature of 50 °C.

According to a particularly preferred embodiment, the radical polymerizable polyacidic polymer has repeating units of the following formula (IV): 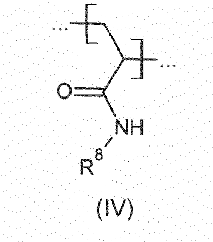 wherein R⁸ represents a group of the following formula (V'): 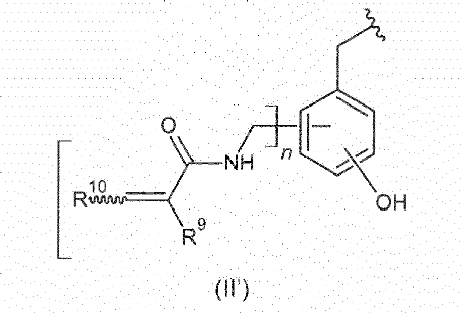 wherein
R⁹ and R¹⁰,
   which may be the same or different, independently represent a hydrogen atom, or a C₁₋₄ alkyl group; preferably R⁹ is a hydrogen atom or a methyl group and R¹⁰ is a hydrogen atom, and
n is an integer of 1 to 3, preferably n is an integer of 1 or 2,
which radical polymerizable polyacidic polymer further comprises acidic repeating units of the following formula (VI): 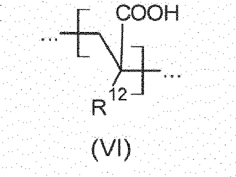 wherein
- R¹²: represents a hydrogen atom, or a C₁₋₄ alkyl group, preferably R¹² represents a hydrogen atom or a methyl group
wherein the molar ratio of repeating units of formula (VI) and repeating units of formula (IV) ([formula (VI)]/[formula (IV)]) is in the range of 100:1 to 5:1, preferably 50:1 to 10:1, and the molecular weight M_{w} is in the range of 20,000 to 150,000, preferably 30,000 to 100,000.

In one embodiment of the present invention, the compound (a) may be (a2) a water-soluble, hydrolysis stable polymerizable crosslinker having at least two polymerizable carbon-carbon double bonds (a2) is termed as "crosslinker (a2)" hereinafter.

Preferably, the crosslinker (a2) is a polymerizable compound of the following formula (XV), which is disclosed in EP2705827 and WO2014040729:

A-L^{c}(B')_{n'} (XV)

wherein
- A: is a group of the following formula (XVI) 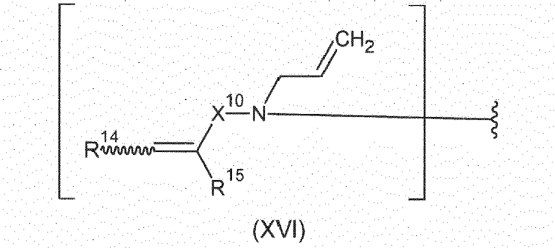
- X¹⁰: is CO, CS, CH₂, or a group [X¹⁰⁰Z¹⁰]ₖ, wherein X¹⁰⁰ is an oxygen atom, a sulfur atom or NH, Z¹⁰ is a straight chain or branched C₁₋₄ alkylene group, and k is an integer of from 1 to 10;
- R¹⁴: is a hydrogen atom,
-COOM¹⁰,
a straight chain or branched C₁₋₁₆ alkyl group which may be substituted by a C₃₋₆ cycloalkyl group, a C₆₋₁₄ aryl or C₃₋₁₄ heteroaryl group, -COOM¹⁰, -PO₃M¹⁰, -O-PO₃M¹⁰₂ or -SO₃M¹⁰,
a C₃₋₆ cycloalkyl group which may be substituted by a C₁₋₁₆ alkyl group, a C₆₋₁₄ aryl or C₃₋₁₄ heteroaryl group, -COOM¹⁰, -PO₃M¹⁰, -O-PO₃M¹⁰₂ or -SO₃M¹⁰,
a C₆₋₁₄ aryl or C₃₋₁₄ heteroaryl group which may be substituted by -COOM¹⁰,-PO₃M¹⁰, -O-PO₃M¹⁰₂ or -SO₃M¹⁰,
- R¹⁵: is a hydrogen atom,
-COOM¹⁰
a straight chain or branched C₁₋₁₆ alkyl group which may be substituted by a C₆₋₁₄ aryl or C₃₋₁₄ heteroaryl group, -COOM¹⁰, -PO₃M¹⁰, -O-PO₃M¹⁰₂ and -SO₃M¹⁰, a C₃₋₆ cycloalkyl group which may be substituted by a C₁₋₁₆ alkyl group, a C₆₋₁₄ aryl or C₃₋₁₄ heteroaryl group, -COOM¹⁰, -PO₃M¹⁰, -O-PO₃M¹⁰₂ or -SO₃M¹⁰, or
a C₆₋₁₄ aryl or C₃₋₁₄ heteroaryl group which may be substituted by -COOM¹⁰,-PO₃M¹⁰, -O-PO₃M¹⁰₂ and -SO₃M¹⁰,
- L^{c}: is a single bond or a linker group;
- B': independently is
a group according to the definition of A,
a group of the following formula (XVII) 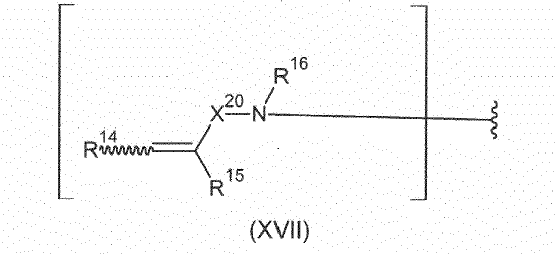 wherein
X²⁰ independently has the same meaning as defined for X¹⁰ in formula(XVI),
R¹⁴ and R¹⁵ are independent from each other and independently have the same meaning as defined for formula (XVI),
R16 is a hydrogen atom,
a straight chain or branched C₁₋₁₆ alkyl group which may be substituted by a C₃₋₆ cycloalkyl group, a C₆₋₁₄ aryl or C₃₋₁₄ heteroaryl group, -COOM¹⁰,-PO₃M¹⁰, -O-PO₃M¹⁰₂ or -SO₃M¹⁰,
a C₃₋₆ cycloalkyl group which may be substituted by a C₁₋₁₆ alkyl group, a C₆₋₁₄ aryl or C₃₋₁₄ heteroaryl group, -COOM¹⁰, -PO₃M¹⁰, -O-PO₃M¹⁰₂ or -SO₃M¹⁰,
a C₆₋₁₄ aryl group which may be substituted by -COOM¹⁰, -PO₃M¹⁰, -O-PO₃ M¹⁰₂ or -SO₃M¹⁰,
a group of the following formula (XVIII) 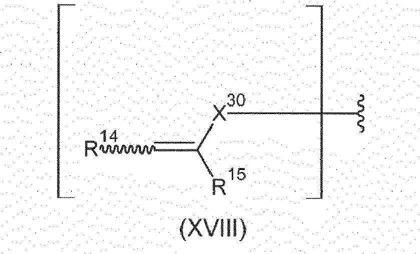 wherein
X³⁰ is CO, -CH₂CO-, CS, or -CH₂CS-,
R¹⁴ and R¹⁵ which are independent from each other and independently have the same meaning as defined for formula (XVI), or
a group [X⁴⁰Z²⁰⁰]ₚE,
wherein
Z²⁰⁰ is a straight chain or branched C₁₋₄ alkylene group,
X⁴⁰ is an oxygen atom, a sulfur atom or NH,
E is a hydrogen atom,
   PO₃M₂,
   a straight chain or branched C₁₋₁₆ alkyl group which may be substituted by a C₃₋₆ cycloalkyl group, a C₆₋₁₄ aryl or C₃₋₁₄ heteroaryl group, -COOM¹⁰, -PO₃M¹⁰, -O-PO₃M¹⁰₂ or -SO₃M¹⁰,
      a C₃₋₆ cycloalkyl group which may be substituted by a C₁₋₁₆ alkyl group, a C₆₋₁₄ aryl or C₃₋₁₄ heteroaryl group, -COOM¹⁰,-PO₃M¹⁰, -O-PO₃M¹⁰₂ or -SO₃M¹⁰,
      a C₆₋₁₄ aryl or C₃₋₁₄ heteroaryl group which may be substituted by -COOM¹⁰, -PO₃M¹⁰, -O-PO₃M¹⁰₂ or -SO₃M¹⁰, and
p^{c} is an integer of from 1 to 10;
and
n' is an integer of from 1 to 4;
wherein M¹⁰ which are independent from each other each represent a hydrogen atom or a metal atom. Preferably, when L^{c} is a single bond, B' cannot be a group according to the definition of A or a group of the formula (XVII).

The following groups are preferred groups of formula (XVI), wherein M¹⁰ is a hydrogen atom or a metal atom:

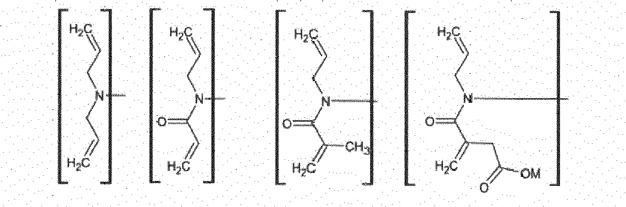

Preferred divalent linker groups may be selected from methylene, ethylene, propylene, butylene and the following divalent groups:

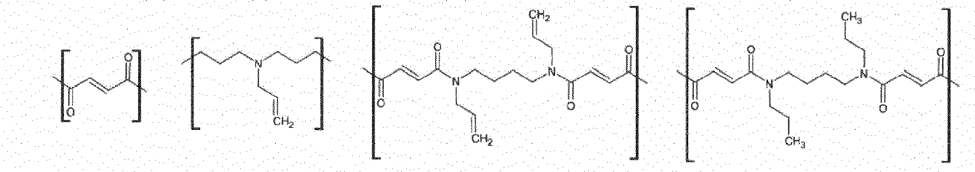

N,N'-(2E)-but-2-en-1,4-diallylbis-[(N-prop-2-en-1) amide and N,N-di(allyl acrylamido) propane are preferred.

Alternatively or additionally, compound (a) may be a crosslinker selected from the group consisting of an alkylenediol dimethylacrylate such as 1,3-butanediol dimethacrylate, 1,4-butanediol dimethacrylate, an alkylenediol divinyl ether such as 1,4-butanediol divinyl ether, di(ethylene glycol) dimethacrylate, di(ethylene glycol) divinyl ether, pentaerythritol diacrylate monostearate, ethylene glycol dimethacrylate, trimetylolpropane trimethacrylate, pentaerythritol triacrylate or triallyl ether, pentaerythritol tetraacrylate and trimetylolpropane triacrylate.

The one or more compounds having a radical polymerizable carbon-carbon double bond may be present in the redox curing dental composition according to the present invention in an amount of from 0.1 wt% to 90 wt% based on the total weight of the dental composition, preferably in an amount of from 1 wt% to 85 wt% based on the total weight of the dental composition, more preferably in an amount of from 2 wt% to 80 wt% based on the total weight of the dental composition, still more preferably of from 5 wt% to 70 wt% based on the total weight of the dental composition, in particular of from 10 wt% to 50 wt% based on the total weight of the dental composition.

In a preferred embodiment of the present invention, wherein the redox curing dental composition is a resin-modified dental cement, the one or more compounds having a radical polymerizable carbon-carbon double bond may be present in the resin-modified dental cement in an amount of from 0.1 to 20, more preferably 1 to 15 even more preferably 2 to 10 percent by weight based on the total weight of the resin-modified dental cement composition. When the one or more compounds having a radical polymerizable carbon-carbon double bond are absent, a long-term mechanical resistance may be low. On the other hand, when the amount of the one or more compounds having a radical polymerizable carbon-carbon double bond exceeds 20 percent of weight, shrinkage of the resin-modified dental cement may occur.

### (b) The redox initiator system

According to the present invention, the redox curing dental composition comprises (b) a redox initiator system.

The redox initiator system comprises a reducing agent which is a compound of the following formula (I): 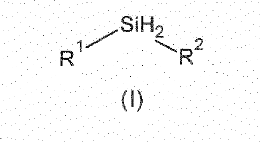 wherein R¹ and R², which may be the same or different, independently represent a C₁₋₁₈ hydrocarbyl group, or R¹ and R² form together with the silicon atom to which they are bonded a 3 to 8-membered silicon-containing alicyclic ring, which groups R¹ and/or R² may be substituted with one to three substituents selected from the group consisting of a C₁₋₆ alkoxy groups, halogen atoms, or a carboxylic acid group; and

In a compound of formula (I), R¹ and R² may be the same or different. According to a preferred embodiment, R¹ and R² are the same. When R¹ and R² are different, R¹ and R² independently represent a C₁₋₁₈ hydrocarbyl group, preferably a C₂₋₁₂ hydrocarbyl group.

The C₁₋₁₈ hydrocarbyl group may be a C₁₋₁₈ aliphatic group, a C₃₋₁₀ cycloaliphatic group, a C₆₋₁₂ aryl group, or a C₇₋₁₈ arylalkyl group. Preferably, the C₁₋₁₈ hydrocarbyl group is a C₁₋₁₈ aliphatic group, C₃₋₁₀ cycloaliphatic group, or a C₆₋₁₂ aryl group, more preferably the C₁₋₁₈ hydrocarbyl group is a C₂₋₁₂ aliphatic group, or a C₆₋₁₂ aryl group, in particular a C₆₋₁₀ aryl group.

Preferably, the hydrocarbyl group is unsubstituted. However, the hydrocarbyl group may be substituted with one to three substituents, preferably one or two substituents, which are selected from the group consisting of a C₁₋₆ alkoxy groups, halogen atoms, or a carboxylic acid group.

According to a preferred embodiment, R¹ and R², which may be the same or different, independently represent an unsubstituted C₁₋₆ alkyl group, or an unsubstituted C₆₋₁₀ aryl group. In a particularly preferred embodiment one of the groups R¹ and R² represent a methyl group and the other group R¹ or R² represents a phenyl group.

In a preferred embodiment, the C₁₋₁₈ hydrocarbon group is a C₁₋₄ aliphatic group, in particular a methyl group.

In a particularly preferred embodiment, the compound of formula (I) represents a compound of the following formula (I*): 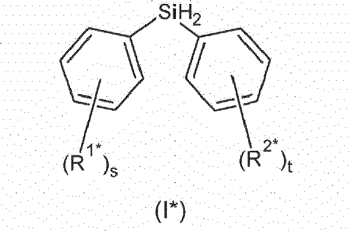 wherein
R^{1*} which may be the same or different when more than one R^{1*} is present, independently represents a C₁₋₆ alkyl group, C₂₋₆ alkenyl group, a C₁₋₆ alkoxy group, a halogen atom, or a carboxylic acid group;
R^{2*} which may be the same or different when more than one R^{2*} is present, independently represents, a C₁₋₆ alkyl group, C₂₋₆ alkenyl group, a C₁₋₆ alkoxy group, a halogen atom, or a carboxylic acid group; or
a substituent R^{1*} and a substituent R^{2*} form together a single bond or a C₁₋₆ alkylene or C₂₋₆ alkenylene group;
s and t, which may be the same or different, represent 0 or an integer of 1 to 5.

In a compound of formula (I*), R^{1*} which may be the same or different when more than one R^{1*} is present, independently represents a C₁₋₆ alkyl group, C₂₋₆ alkenyl group, a C₁₋₆ alkoxy group, a halogen atom, or a carboxylic acid group. Preferably, R^{1*} is a C₁₋₆ alkyl group, C₂₋₆ alkenyl group, a C₁₋₆ alkoxy group, or a halogen atom, more preferably a C₁₋₆ alkyl group, a C1-6 alkoxy group, or a halogen atom, even more preferably a C₁₋₆ alkyl group or a C₁₋₆ alkoxy group, in particular a C₁₋₆ alkyl group. In a specifically preferred embodiment, R^{1*} is a C1-6 alkyl group, more preferred R^{1*} is a C₁₋₄ alkyl group, even more preferred R^{1*} is a C₂₋₄ alkyl group, in particular R^{1*} is a C₄ alkyl group. In a particularly preferred embodiment R^{1*} is the following group:

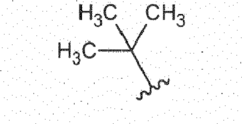

In a compound of formula (I*), R^{2*} which may be the same or different when more than one R^{2*} is present, independently represents a C₁₋₆ alkyl group, C₂₋₆ alkenyl group, a C₁₋₆ alkoxy group, a halogen atom, or a carboxylic acid group. Preferably, R^{2*} is a C₁₋₆ alkyl group, C₂₋₆ alkenyl group, a C₁₋₆ alkoxy group, or a halogen atom, more preferably a C₁₋₆ alkyl group, a C₁₋₆ alkoxy group, or a halogen atom, even more preferably a C₁₋₆ alkyl group or a C₁₋₆ alkoxy group, in particular a C₁₋₆ alkyl group. In a specifically preferred embodiment, R^{4*} is a C₁₋₆ alkyl group, more preferred R^{2*} is a C₁₋₄ alkyl group, even more preferred R^{2*} is a C₂₋₄ alkyl group, in particular R^{2*} is a C₄ alkyl group. In a particularly preferred embodiment R^{2*} is the following group:

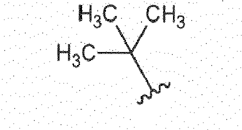

In one embodiment, a substituent R^{1*} and a substituent R^{2*} form together a single bond or a C₁₋₆ alkylene or C₂₋₆ alkenylene group, preferably a substituent R^{1*} and a substituent R^{2*} form together a single bond or a C₁₋₆ alkylene group, more preferably a substituent R^{1*} and a substituent R^{2*} form together a single bond.

In a compound of formula (I*), s and t which may be the same or different, represent 0 or an integer of 1 to 5, more preferably s and t represent 0 or an integer of from 1 to 3, even more preferably s and t represent 0 or an integer of from 1 to 2, still more preferably s and t represent 0 or 1, in particular s and t represent 0.

In a particularly preferred embodiment, the compound of formula (I*) is the following compound:

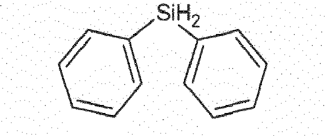

The redox initiator system (b) comprises the reducing agent which is a compound of formula (I) generally in an amount which is sufficient to provide a redox initiator system which is capable of providing a suitable initiation rate for the radical polymerization of the one or more compounds having a radical polymerizable carbon-carbon double bond. In a preferred embodiment, the redox initiator system (b) comprises the reducing agent which is a compound of formula (I) in an amount of from 0.05 wt% to 2.5 wt%, more preferably in an amount of from 0.1 wt% to 2 wt%, even more preferably of from 0.1 wt% to 1.5 wt%, still more preferably of from 0.1 wt% to 1.0 wt%, still even more preferably of from 0.2 wt% to 1.0 wt%, in particular 0.2 wt% to 1.0 wt% based on the total weight of the dental composition.

The redox initiator system further comprises an oxidizing agent adapted to oxidize the reducing agent of formula (I) in the absence of light for generating a radical capable of initiating radical polymerization of the one or more compounds having a radical polymerizable carbon-carbon double bond.

According to a preferred embodiment, the oxidizing agent has a reduction potential in the range of from -0.15 to -1.2 V as determined by cyclic voltammetry, more preferably the oxidizing agent has a reduction potential in the range of from -0.2 to -1.1 V as determined by cyclic voltammetry.

The reduction potentials (E_{red} vs SCE) are determined at 23 °C by cyclic voltammetry in a DCM solution containing tetrabutylammonium hexafluorophosphate (Aldrich) as a supporting electrolyte. A platinum electrode is used as a working electrode and a saturated calomel electrode (SCE) was used as a reference electrode.

According to a preferred embodiment, the oxidizing agent has a storage stability so that a 1 mol/liter aqueous solution of the oxidizing agent decomposes in an amount of less than 10 % when stored at 50 °C for four weeks.

Preferably, the oxidizing agent contains a metal cation or a cation of the following formula (II): 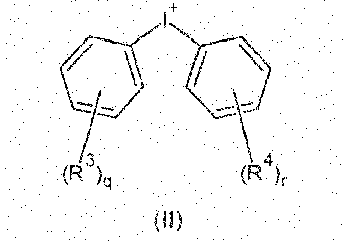 wherein
R³ which may be the same or different when more than one R³ is present, independently represents a C₁₋₆ alkyl group, C₂₋₆ alkenyl group, a C₁₋₆ alkoxy group, a halogen atom, or a carboxylic acid group;
R⁴ which may be the same or different when more than one R⁴ is present, independently represents, a C₁₋₆ alkyl group, C₂₋₆ alkenyl group, a C₁₋₆ alkoxy group, a halogen atom, or a carboxylic acid group; or
a substituent R³ and a substituent R⁴ form together a single bond or a C₁₋₆ alkylene or C₂₋₆ alkenylene group;
q and r, which may be the same or different, represent 0 or an integer of 1 to 5.

In one embodiment of the present invention, the oxidizing agent contains a metal cation. In one embodiment of the present invention, the metal cation is selected from the group consisting of cobalt, iron, cerium, copper and manganese. Preferably, the metal cation is copper. Oxidizing agents containing metal cations include salts of transition metals such as cobalt (III) chloride and ferric chloride, and cerium (IV) sulfate, perboric acid metal salts, permanganic acid metal salts, perphosphoric acid metal salts, and mixtures thereof. In a particularly preferred embodiment, the oxidizing agent contains a Cu²⁺ cation. In particular the oxidizing agent is the following compound.

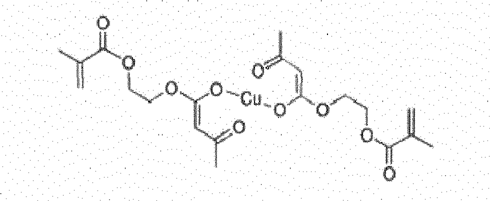

In a cation of formula (II), R³ which may be the same or different when more than one R³ is present, independently represents a C₁₋₆ alkyl group, C₂₋₆ alkenyl group, a C₁₋₆ alkoxy group, a halogen atom, or a carboxylic acid group. Preferably, R³ is a C₁₋₆ alkyl group, C₂₋₆ alkenyl group, a C₁₋₆ alkoxy group, or a halogen atom, more preferably a C₁₋₆ alkyl group, a C₁₋₆ alkoxy group, or a halogen atom, even more preferably a C₁₋₆ alkyl group or a C₁₋₆ alkoxy group, in particular a C₁₋₆ alkyl group. In a specifically preferred embodiment, R¹ is a C₁₋₆ alkyl group, more preferred R³ is a C₁₋₄ alkyl group, even more preferred R³ is a C₂₋₄ alkyl group, in particular R³ is a C₄ alkyl group. In a particularly preferred embodiment R¹ is the following group:

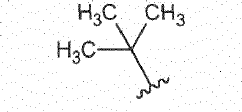

In a cation of formula (II), R⁴ which may be the same or different when more than one R⁴ is present, independently represents a C₁₋₆ alkyl group, C₂₋₆ alkenyl group, a C₁₋₆ alkoxy group, a halogen atom, or a carboxylic acid group. Preferably, R⁴ is a C₁₋₆ alkyl group, C₂₋₆ alkenyl group, a C₁₋₆ alkoxy group, or a halogen atom, more preferably a C₁₋₆ alkyl group, a C₁₋₆ alkoxy group, or a halogen atom, even more preferably a C₁₋₆ alkyl group or a C₁₋₆ alkoxy group, in particular a C₁₋₆ alkyl group. In a specifically preferred embodiment, R⁴ is a C₁₋₆ alkyl group, more preferred R⁴ is a C₁₋₄ alkyl group, even more preferred R⁴ is a C₂₋₄ alkyl group, in particular R⁴ is a C₄ alkyl group. In a particularly preferred embodiment R⁴ is the following group:

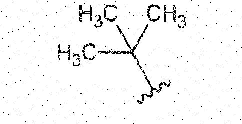

In one embodiment, a substituent R³ and a substituent R⁴ form together a single bond or a C₁₋₆ alkylene or C₂₋₆ alkenylene group, preferably a substituent R³ and a substituent R⁴ form together a single bond or a C₁₋₆ alkylene group, more preferably a substituent R³ and a substituent R³ form together a single bond.

In a cation of formula (II), q and r which may be the same or different, represent 0 or an integer of 1 to 5, more preferably q and r represent 0 or an integer of from 1 to 3, even more preferably q and r represent 0 or an integer of from 1 to 2, still more preferably q and r represent 0 or 1, in particular q and r represent 1.

In a preferred embodiment, in a cation of formula (II) q and r represent 1 and R³ and R⁴ independently represent a C₁₋₆ alkyl group.

In a preferred embodiment of the present invention, the cation of formula (II) is selected from the cations of the following formulae: 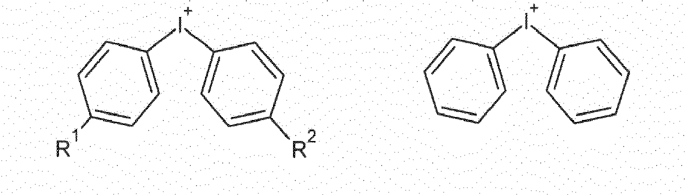 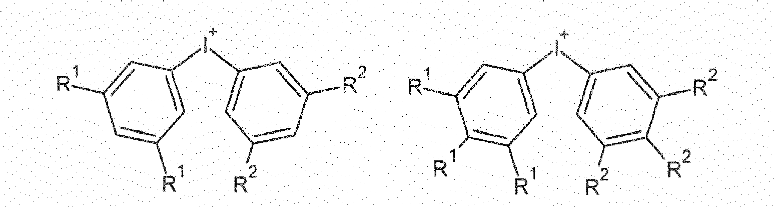 wherein R³ and R⁴ are defined as they are defined in formula (II).

In a specifically preferred embodiment the cation of formula (II) is the following cation:

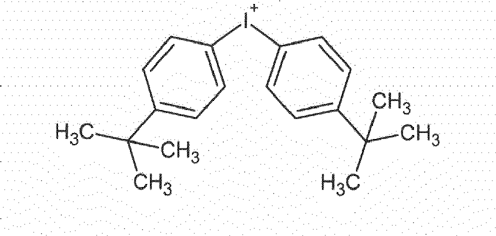

In the preferred embodiment of the present invention, wherein the oxidizing agent contains a cation of the formula (II), the oxidizing agent may further contain any anion which is known in the art. Preferably, the anion may be selected from hexafluoroantimonate, trifluoromethylsulfate, hexafluorophosphate, tetrafluoroborate, hexafluoroarsenate, and tetraphenylborate, more preferably the anion may be selected from hexafluoroantimonate, hexafluorophosphate, and tetrafluoroborate, even more preferably the anion may be selected from hexafluoroantimonate and hexafluorophosphate, in particular the anion is hexafluorophosphate.

In an especially preferred embodiment, the oxidizing agent containing a cation of formula (II) is the following compound.

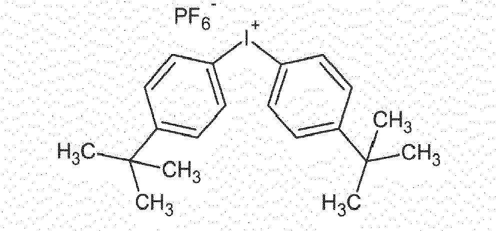

The redox initiator system (b) comprises the oxidizing agent, preferably containing a metal cation or a cation of formula (II), generally in an amount which is sufficient to generate a sufficient number of radicals to provide a suitable initiation rate for the radical polymerization of the one or more compounds having a radical polymerizable carbon-carbon double bond. In a preferred embodiment, the redox initiator system (b) comprises the oxidizing agent containing a metal cation or a cation of formula (II) in an amount of from 0.05 wt% to 2.5 wt%, more preferably in an amount of from 0.1 wt% to 2 wt%, even more preferably of from 0.1 wt% to 1.5 wt%, still more preferably of from 0.1 wt% to 1.0 wt%, still even more preferably of from 0.2 wt% to 1.0 wt%, in particular 0.2 wt% to 1.0 wt% based on the total weight of the dental composition.

According to a preferred embodiment, the redox initiator system (b) further comprises a phosphine compound of the following formula (III): 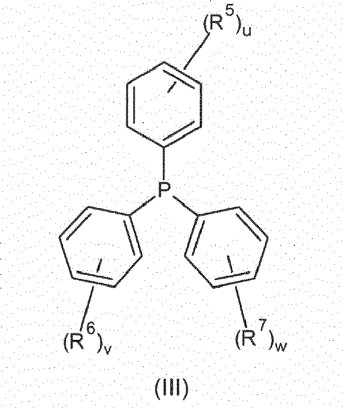 wherein
R⁵ which may be the same or different when more than one R⁵ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group;
R⁶ which may be the same or different when more than one R⁶ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group;
R⁷ which may be the same or different when more than one R⁷ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group; or
   two substituents selected from R⁵, R⁶ and R⁷ form together a single bond or a C₁₋₆ alkylene or C₂₋₆ alkenylene group;
u, v and w, which may be the same or different, represent 0 or an integer of 1 to 5.

In a phosphine compound of formula (III), R⁵ which may be the same or different when more than one R⁵ is present, independently represents a C₁₋₆ alkyl group, C₂₋₆ alkenyl group, a C_{1- 6} alkoxy group, a C₁₋₆ alkylamino group, a di-(C₁₋₆ alkyl)amino group a halogen atom, or a carboxylic acid group. Preferably, R⁵ represents a C₂₋₆ alkenyl group, a C₁₋₆ alkylamino group, a di-(C₁₋₆ alkyl)amino group, or a carboxylic acid group, more preferably R⁵ represents a C₂₋₆ alkenyl group, a di-(C₁₋₆ alkyl)amino group, or a carboxylic acid group, even more preferably R⁵ is a di-(C₁₋₆ alkyl)amino group, or a carboxylic acid group, in particular R⁵ is a di-(C₁₋₆ alkyl)amino group. In a preferred embodiment, wherein R⁵ is a di-(C₁₋₆ alkyl)amino group, R⁵ preferably is a di-(C₁₋₄ alkyl)amino group, more preferably a di-(C₁₋₃ alkyl)amino group, in particular a di-methylamino group.

In a phosphine compound of formula (III), R⁶ which may be the same or different when more than one R⁶ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group. Preferably, R⁶ represents a C₂₋₆ alkenyl group, a C₁₋₆ alkylamino group, a di-(C₁₋₆ alkyl)amino group, or a carboxylic acid group, more preferably R⁶ represents a C₂₋₆ alkenyl group, a di-(C₁₋₆ alkyl)amino group, or a carboxylic acid group, even more preferably R⁶ is a di-(C₁₋₆ alkyl)amino group, or a carboxylic acid group, in particular R⁶ is a di-(C₁₋₆ alkyl)amino group. In a preferred embodiment, wherein R⁶ is a di-(C₁₋₆ alkyl)amino group, R⁶ preferably is a di-(C₁₋₄ alkyl)amino group, more preferably a di-(C₁₋₃ alkyl)amino group, in particular a di-methylamino group.

In a phosphine compound of formula (III), R⁷ which may be the same or different when more than one R⁷ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group. Preferably, R⁷ represents a C₂₋₆ alkenyl group, a C₁₋₆ alkylamino group, a di-(C₁₋₆ alkyl)amino group, or a carboxylic acid group, more preferably R⁷ represents a C₂₋₆ alkenyl group, a di-(C₁₋₆ alkyl)amino group, or a carboxylic acid group, even more preferably R⁷ is a di-(C₁₋₆ alkyl)amino group, or a carboxylic acid group, in particular R⁷ is a di-(C₁₋₆ alkyl)amino group. In a preferred embodiment, wherein R⁷ is a di-(C₁₋₆ alkyl)amino group, R⁷ preferably is a di-(C₁₋₄ alkyl)amino group, more preferably a di-(C₁₋₃ alkyl)amino group, in particular a di-methylamino group.

In one embodiment, two substituents selected from R⁵, R⁶ and R⁷ form together a single bond or a C₁₋₆ alkylene or C₂₋₆ alkenylene group, preferably two substituents selected from R⁵, R⁶ and R⁷ form together a single bond or a C₁₋₆ alkylene group, more preferably two substituents selected from R⁵, R⁶ and R⁷ form together a single bond.

In a phosphine of formula (III) u, v and w, which may be the same or different, represent 0 or an integer of 1 to 5. Preferably, u, v, and w represent 0 or an integer of from 1 to 3, more preferably u, v, and w represent 0 or an integer of from 1 to 2, and even more preferably u, v, and w represent 0 or 1. Furthermore, in a specifically preferred embodiment, u+v+w = 1.

In a particularly preferred embodiment, a phosphine of formula (III) is selected from the following compounds:

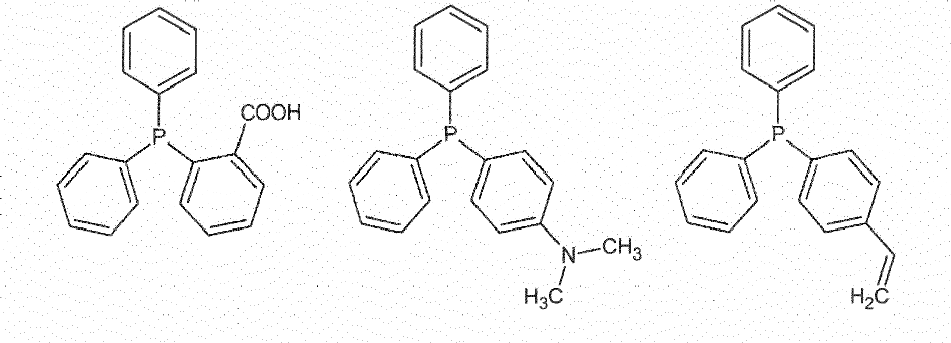

In an especially preferred embodiment, a phosphine of formula (III) is the following compound:

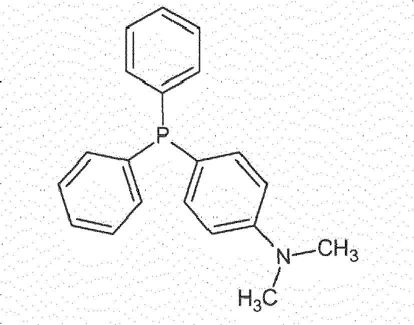

The redox initiator system (b) comprises the phosphine compound of formula (III) generally in an amount which is sufficient to provide a redox initiator system which is capable of providing a suitable initiation rate for the radical polymerization of the one or more compounds having a radical polymerizable carbon-carbon double bond. In a preferred embodiment, the redox initiator system (b) comprises the phospine compound of formula (III) in an amount of from 0.05 wt% to 2.5 wt%, more preferably in an amount of from 0.1 wt% to 2 wt%, even more preferably of from 0.1 wt% to 1.5 wt%, still more preferably of from 0.1 wt% to 1.0 wt%, still even more preferably of from 0.2 wt% to 1.0 wt%, in particular 0.2 wt% to 1.0 wt% based on the total weight of the dental composition.

The present invention provides a redox initiator system as defined herein. Preferably, the present invention provides a redox initiator system comprising a compound of formula (I), and a compound of formula (II). More preferably, the present invention provides a redox initiator system comprising a compound of formula (I), a compound of formula (II), and a compound of formula (III).

Furthermore, the present invention provides a use of a redox initiator system as defined herein.

Moreover, the present invention provides a redox initiator system as defined herein for use in the treatment or prevention of endodontic disease.

### Further optional Components

### The photosensitizer

The redox initiator system is an essential feature of the present invention and may be the only initiator system of the dental composition. However, in a specific embodiment the redox curing dental composition according to the present invention comprises a dual cure initiator system containing the redox initiator system and at least one photosensitizer.

The redox curing dental composition according to the present invention may comprise any photosensitizer suitable for dental compositions and/or known in the art. Preferably, the redox curing dental composition according to the present invention comprises a photosensitizer which has an absorption maximum in the range of 400 to 800 nm.

Suitable photosensitizers are Norrish type I and Norrish type II photosensitizers.

The term "Norrish type I" refers to a photosensitizer undergoing excitation by energy absorption with subsequent decomposition of the compound into one or more radicals.

The term "Norrish type II" refers to a photosensitizer undergoing excitation, and the excited photosensitizer interacts with a second compound, such as a coinitiator, an electron donor, or a sensitizer, by either energy transfer or a redox reaction to form free radicals from any of the compounds.

Phosphine oxide photosensitizers may have a functional wavelength range of about 380 nm to about 450 nm, which include acyl and bisacyl phosphine oxides such as those described in US 4,298,738, US 4,324,744 US and 4,385,109 and EP 0 173 567. Specific examples of the acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphonate, and 2,4,6-trimethylbenzoylethoxyphenylphosphine oxide. Commercially available phosphine oxide photosensitizers capable of free-radical initiation when irradiated at wavelength ranges of greater than about 380 nm to about 450 nm include bis(2,4,6-trimethylbenzoyl)phenyl phosphine oxide (IRGACURE 819), bis(2,6-dimethoxybenzoyl)-(2,4,4-trimethylpentyl) phosphine oxide (CGI 403), a 25:75 mixture, by weight, of bis(2,6-dimethoxybenzoyl)-2,4,4-trimethylpentyl phosphine oxide and 2-hydroxy-2-methyl-1-phenylpropan-1-one (IRGACURE 1700), a 1:1 mixture, by weight, of bis(2,4,6-trimethylbenzoyl)phenyl phosphine oxide and 2-hydroxy-2-methyl-1-phenylpropane-1-one (DAROCUR 4265), and ethyl 2,4,6-trimethylbenzylphenyl phosphinate (LUCIRIN LR8893X). Typically, the phosphine oxide photosensitizer is present in the composition in catalytically effective amounts, such as from 0.1 percent by weight to 5.0 percent by weight, based on the total weight of the composition.

Suitable Si- or Ge-acyl compounds preferably have the following formula (XIX):

X¹-R¹⁷ (XIX)

wherein
- X¹: is a group of the following formula (XX): 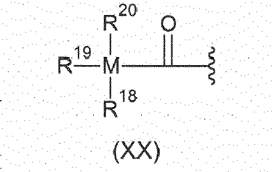 wherein
M is Si or Ge;
R¹⁸ represents a substituted or unsubstituted hydrocarbyl or hydrocarbylcarbonyl group;
R¹⁹ represents a substituted or unsubstituted hydrocarbyl or hydrocarbylcarbonyl group;
R²⁰ represents a substituted or unsubstituted hydrocarbyl group; and
- R¹⁷: i) has the same meaning as X¹, whereby the compound of formula (XIX) may be symmetrical or unsymmetrical; or
ii) is a group of the following formula (XXI): 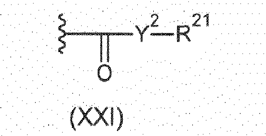
wherein
Y² represents a single bond, an oxygen atom or a group NR', wherein R' represents a substituted or unsubstituted hydrocarbyl group;
R²¹ represents a substituted or unsubstituted hydrocarbyl group, a trihydrocarbylsilyl group, a mono(hydrocarbyl-carbonyl)dihydrocarbylsilyl group or a di(hydrocarbyl-carbonyl)mono-hydrocarbylsilyl group.

In connection with the Si- or Ge-acyl compound of formula (XIX), the term "substituted" as used herein means that R¹⁸, R¹⁹, R²⁰, R²¹ and R' may be substituted by a substituent selected from the group consisting of halogen atoms, a nitro group, a cyano group, a hydroxy group, an amino group, C₁₋₆ alkyl groups, C₁₋₆ alkoxy groups and a -NR^{x}R^{y} group wherein R^{x} and R^{y} independently from each other represent a C₁₋₆ alkyl group. Here, illustrative of the halogen atoms can be fluorine, chlorine, bromine and iodine. The C₁₋₆ alkyl groups are, for example, methyl, ethyl, n-propyl, isopropyl and n-butyl. Illustrative of the C₁₋₆ alkoxy groups are, for example, methoxy, ethoxy and propoxy. The alkyl moieties in these substituents may be linear, branched or cyclic. Preferably, the substituent is selected from a chlorine atom, a nitro group, a C₁₋₄ alkoxy group and a -NR^{x}R^{y} group wherein R^{x} and R^{y} independently from each other represent a C₁₋₄ alkyl group.

If R¹⁸, R¹⁹ and R²⁰ are substituted, then it is preferred that they are substituted with 1 to 3 substituents, more preferably with 1 substituent.

In the compound of formula (XIX), moieties R¹⁸, R¹⁹ and R²⁰ may be defined as follows: R¹⁸ and R¹⁹ independently from each other represent a substituted or unsubstituted hydrocarbyl or hydrocarbylcarbonyl group, and R²⁰ represents a substituted or unsubstituted hydrocarbyl group.

The hydrocarbylcarbonyl groups of R¹⁸ and R¹⁹ represent acyl groups (R_{org}-(C=O)-) in which the organic residue R_{org} is a hydrocarbyl residue as defined above.

Compound of formula (XIX) may contain one or two hydrocarbylcarbonyl groups, that is either one of R¹⁸ or R¹⁹ is a hydrocarbylcarbonyl group, or both R¹⁸ and R¹⁹ are hydrocarbylcarbonyl groups. Preferably, compound of formula (XIX) contains one hydrocarbylcarbonyl group.

Preferably, the hydrocarbylcarbonyl group is an arylcarbonyl group, more preferably a benzoyl group.

Preferably, R¹⁸ and R¹⁹ are independently selected from the group consisting of a straight chain or branched C₁₋₆ alkyl group, and a phenyl or benzoyl group which may optionally be substituted by one to three substitutents selected from halogen atoms, a nitro group, a C₁₋₄ alkoxy group and a -NR^{x}R^{y} group wherein R^{x} and R^{y} independently from each other represent a C₁₋₄ alkyl group, and R²⁰ is a straight chain or branched C₁₋₆ alkyl group or a phenyl group.

Most preferably, R¹⁸ and R¹⁹ are independently selected from the group consisting of a straight chain or branched C₁₋₄ alkyl group, and a phenyl or benzoyl group which may optionally be substituted with one substituent selected from the group consisting of selected from a halogen atom, a nitro group, a C₁₋₄ alkoxy group and a -NR^{x}R^{y} group wherein R^{x} and R^{y} independently from each other represent a C₁₋₄ alkyl group, and R²⁰ is a straight chain or branched C₁₋₄ alkyl group.

In the compound of formula (XIX), R¹⁷ may have the same meaning as X¹, whereby the compound of formula (XIX) may be symmetrical or unsymmetrical. Alternatively, R¹⁷ may represent a substituted or unsubstituted hydrocarbyl group, or a group of formula (XXI). Preferably, if R¹⁷ has the same meaning as X¹, then compound of formula (XIX) is unsymmetrical. If R¹⁷ represents a substituted or unsubstituted hydrocarbyl group, then the hydrocarbyl group has the same meaning as defined above for R¹⁸ and is independently selected therefrom.

In the group of formula (XXI) of compound of formula (XIX), R²¹ represents a substituted or unsubstituted hydrocarbyl group, a trihydrocarbylsilyl group, a mono(hydrocarbylcarbonyl)-dihydrocarbylsilyl group or a di(hydrocarbylcarbonyl)monohydrocarbylsilyl group.

If R²¹ of formula (XXI) is a trihydrocarbylsilylgroup, a mono(hydrocarbylcarbonyl)-dihydrocarbylsilyl group or a di(hydrocarbylcarbonyl)monohydrocarbylsilyl group, each of the hydrocarbyl and hydrocarbylcarbonyl groups has the same meaning as defined for R¹⁸, R¹⁹ and R²⁰ and is independently selected therefrom.

In formula (XXI), R' has the same meaning as defined for R²¹ and is independently selected therefrom.

For example, compounds of formula (XIX) wherein R¹⁷ has the same meaning as X¹ and which are symmetrical may be have the following structural formulae:

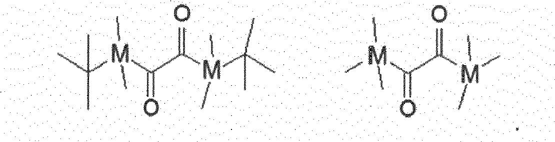

For example, compounds of formula (XIX) wherein R¹⁷ represents a group of formula (XXI) wherein Y² is a bond, an oxygen atom or a NR' group, and R²¹ represents a substituted or unsubstituted hydrocarbyl group may have the following structural formulae:

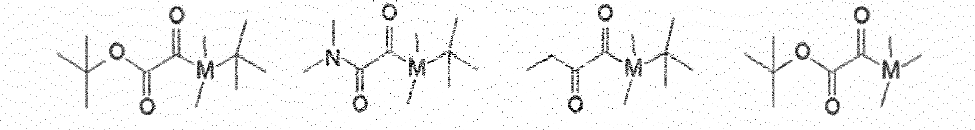

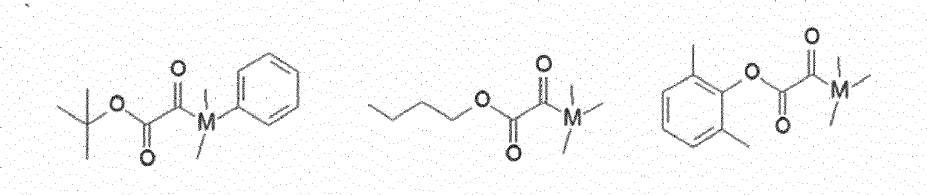

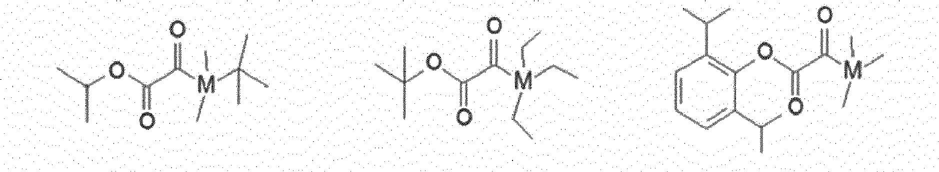

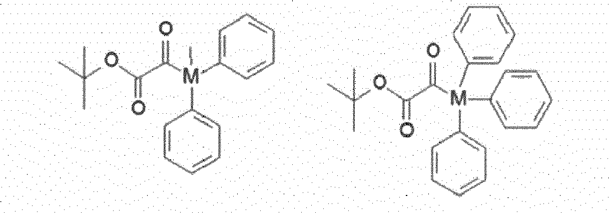

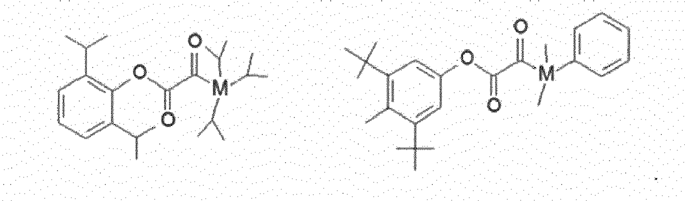

For example, compounds of formula (XIX) wherein R¹⁷ represents a group of formula (XXI) wherein R²¹ represents a trihydrocarbylsilyl group have the following structural formulae:

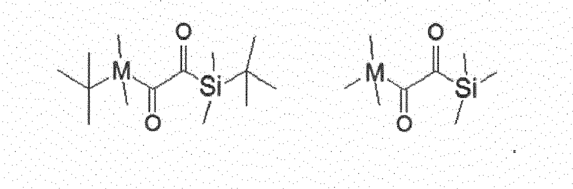

Preferably, compound of formula (XIX) is selected from the group consisting of: 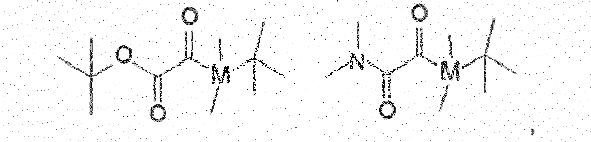 wherein compounds of formula (XIX) with M = Si are particularly preferred.

More preferably, compound of formula (XIX) has the following structural formula: 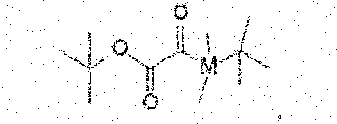 wherein it is particularly preferred that M = Si. That is, tert-butyl (tert-butyldimethylsilyl)-glyoxylate) (DKSi) is particularly preferred.

In case the redox curing dental composition is in the form of an acidic composition, that is a composition having a pH of less than 7, depending on the composition's pH level, it is preferred to select compounds of formula (XIX) with the proviso that they do not contain ester groups, or at least only ester groups which do not significantly hydrolyse in aqueous media at pH 3 at room temperature within one month. Thereby, an advantageous stability of an acidic dental composition, that is a composition having a pH of less than 7, in terms of shelf-life stability of the uncured redox curing dental composition as well as stability after curing in the mouth of a patient is ensured. Therefore, for acidic dental compositions, particularly preferred are compounds of formula (XIX) excluding R¹⁷ being a group of formula (XIX) in which Y² is an oxygen atom.

Furthermore, since the acylsilyl moiety (-C(=O)-Si-) might be sensitive to basic conditions, that is a pH higher than 7, it is preferred to suitably select a pH value of the composition being higher than 7 with the proviso that the acylsilyl moiety is not cleaved in aqueous media at the selected basic pH at room temperature within one month.

The compound of the formula (XIX) may be a known compound which is commercially available or a may be prepared according to published procedures, as described for example in WO 2017/060459 A1.

Suitable Norrish type II photosensitizers may be selected from the group consisting of camphorquinone, benzil, 2,2'-3 3'- and 4,4'-dihydroxylbenzil, 2,3-butanedione, 2,3-pentanedione, 2,3-hexanedione, 3,4-hexanedione, 2,3-heptanedione, 3,4-heptanedione, 2,3-octanedione, 4,5-octanedionefuril, biacetyl, 1,2-cyclohexanedione, 1,2-naphthaquinone, and acenaphthaquinone. Camphorquinone is preferred.

Preferably, irrespective whether Norrish type I or II, the photosensitizer is a 1,2-diketone, even more preferably camphor quinone or a Si- or Ge-acyl compound of formula (XIX), yet even more preferably camphor quinone or DKSi, and most preferably camphor quinone.

### The particulate filler

The redox curing dental composition according to the present invention may further comprise a particulate filler.

The particulate filler usually has an average particle size of from 0.005 to 100 µm, preferably of from 0.01 to 40 µm as measured, for example, by using electron microscopy or by using a conventional laser diffraction particle sizing method as embodied by a MALVERN Mastersizer S or MALVERN Mastersizer 2000 apparatus. The particulate filler may be a monomodal or multimodal when the particular filler is a mixture of two or more radiopaque particulate fractions having different average particle sizes. The particulate filler may also be a mixture of particles of different chemical composition.

In a preferred embodiment, the filler according to the present invention is a radiopaque particulate filler. The radiopaque particulate filler may contain high atom number elements selected from zinc, ytterbium, yttrium, gadolinium, zirconium, strontium, tungsten, tantalum, thorium, niobium, barium, bismuth, molybdenum and lanthanum metals, alloys thereof, organometallic complexes thereof, oxides, sulfates, carbonates, halides, oxy-halides, subnitrates, tungstates and carbides thereof, iodine and inorganic iodides, either singly or in combination. In a preferred embodiment, the radiopaque particulate filler is selected from any of bismuth trioxide, bismuth carbonate, bismuth oxy-chloride, bismuth subnitrate, zirconium oxide, barium sulfate, barium tungstate and calcium tungstate, either singly or in combination. In an even more preferred embodiment, the radiopaque particulate filler is selected from barium tungstate and calcium tungstate, either singly or in combination. Preferably the radiopaque particulate filler is calcium tungstate.

The redox curing dental composition according to the present invention preferably comprises 1 to 85 percent by weight, more preferably 40 to 85 percent by weight, even more preferably 40 to 70 percent by weight, of the radiopaque particulate filler, based on the weight of the entire composition.

The viscosity and thixotropicity of the uncured as well as the physical properties of the cured compositions may be controlled by varying the sizes and surface areas of the particulate filler.

The filler may be surface treated with one or more silanating agents. Preferred silanating agents include those having at least one polymerizable double bond and at least one group that easily hydrolyses with water. Examples of such agents include 3-methacryloxypropyltrimethoxysilane, 3-methacryloxypropyldimethoxy-monochlorosilane, 3-methacryloxypropyldichloromonomethoxysilane, methacryloxypropyltri-chlorosilane, 3-methacryloxypropyldichloromonomethyl-silane, 3-methacryloxypropylmonochlorodimethylsilane, or 2,3-epoxypropyltrimethoxysilane, aminopropyltrimethoxysilane, mercaptopropyltrimethoxysilane and mixtures thereof.

In a particularly preferred embodiment, the redox curing dental composition according to the present invention comprises a reactive particulate filler. The redox curing dental composition may comprise one or a mixture of two or more reactive particulate fillers.

Any granular component being reactive with a polyacidic polymer in a cement reaction may be used as the reactive particulate filler.

Preferably, the reactive particulate filler is one or a mixture of two or more metal oxides, most preferably a glass, i.e. an amorphous solid mixture of metal oxides.

The reactive particulate filler glass is obtainable by transforming a solid mixture of metal oxides by a thermal melt process into a glass followed by milling, which glass is capable of reacting with a polyacidic polymer in a cement reaction.

Any conventional reactive dental glass may be used as reactive particulate filler. Specific examples of particulate reactive glasses are selected from calcium alumino silicate glass, calcium alumino fluorosilicate glass, calcium aluminumfluoroborosilicate glass, strontium aluminosilicate glass, strontium aluminofluorosilicate glass, strontium aluminofluoroborosilicate glass, or ion-leachable glasses, e.g. as described in US-A 3,655,605, US-A 3,814,717, US-A 4,143,018, US-A 4,209,434, US-A 4,360,605 and US-A 4,376,835.

Alternatively or additionally, reactive metal oxides such as zinc oxide and/or magnesium oxide may be used in glass and/or crystalline form as reactive particulate filler.

Preferably, the reactive particulate filleris a glass comprising:
1) 20 to 45% by weight of silica,
2) 20 to 40% by weight of alumina,
3) 20 to 40% by weight of strontium oxide,
4) 1 to 10% by weight of P₂O₅, and
5) 3 to 25% by weight of fluoride.

The present redox curing dental composition preferably comprises 20 to 90 percent by weight of the reactive particulate filler, more preferably 30 to 85 percent by weight, most preferably 20 to 80 percent by weight based on the total weight of the composition.

The reactive particulate filler may be an agglomerated reactive particulate filler which is obtainable by agglomerating a reactive particulate filler in the presence of a modified polyacid and/or polymerizable resin such as (meth)acryloyl monomers. The particle size of the agglomerated reactive particulate filler may be adjusted by suitable size-reduction processes such as milling.

The reactive particulate filler may be surface modified by a surface modifying agent. Preferably, the surface modifying agent is a silane. A silane provides a suitable hydrophobicity to the reactive particulate filler, which allows for an advantageous, homogeneous admixture with organic components of the dental composition. The reactive particulate filler may have silane coupling agent(s) on its surface, for example in the form of a coating at least partly, and preferably fully covering the surface of the reactive particulate filler.

### Further additional optional components

The dental composition according to the present invention may comprise additional optional components such as solvents, free radical scavengers such as 4-methoxyphenol, polymerization inhibitors, surfactants (such as to enhance solubility of an inhibitor e. g., polyoxyethylene), coupling agents to enhance reactivity of fillers e.g. 3-(trimethoxysilyl) propyl methacrylate, rheology modifiers, stabilizers, and/or pigments.

Suitable solvents may be selected from water, alcohols such as methanol, ethanol, propanol (n-, i-), butanol (n-, iso-, tert.-), and ketones such as acetone.

The redox curing dental composition of the present invention may preferably comprise a solvent in an amount of 5 to 75 percent by weight based on the total weight of the dental composition.

Preferably, in the redox curing dental composition of the present invention, water is present in an amount from about 0 wt% to about 40 wt%, more preferably 1.0 wt% to 30 wt%, and most preferably 2.0 wt% to 25 wt% based on the total weight of the dental composition. This preferred amount of water is particularly suitable for a redox curing dental composition in the form of a resin-modified dental cement.

The term "stabilizer" as used herein means any compound capable of preventing polymerizable compounds contained in the redox curing dental composition from spontaneous polymerization during storage. However, the stabilizer does not disturb or prevent intended polymerisation curing of the redox curing dental composition during application.

For example, the stabilizer may be a conventional stabilizer selected from the group consisting of hydroquinone, hydroquinone monomethylether, tert-butyl-hydroquinone, tert-butylhydroxyanisol, propyl gallate and 2,6-di-tert-butyl-p-cresol. From these conventional stabilizers, 2,6-di-tert-butyl-p-cresol is preferred. However, suitable stabilizers may also be selected from reducing agents such as vitamin C, inorganic sulfides and polysulfides and the like.

The redox curing dental composition according to the invention may contain the stabilizer in an amount of 0.001 to 1 percent by weight, preferably 0.005 to 0.8 percent by weight based on the total weight of the composition. When the amount of the stabilizer is below the above indicated lower limit of 0.001, then storage stability of the redox curing dental composition might be insufficient, since the amount of stabilizer is too small to provide a stabilizing effect. However, when the amount of stabilizer is above the maximum threshold of 1 percent by weight, then the applicability of the redox curing dental composition might be negatively affected, since higher amounts of stabilizer may disturb or even substantially prevent intended polymerization curing of the redox curing dental composition during application.

The present invention provides a redox curing dental composition as defined herein, wherein the redox curing dental composition is selected from the group of a resin-modified dental cement, and a dental bonding agent. Preferably, the redox curing dental composition according to the present invention is a resin-modified dental cement.

Furthermore, the present invention provides a redox curing dental composition which is a two part paste-paste composition comprising a first paste containing the reducing agent (i), and a second paste containing the oxidizing agent (ii). Moreover, the present invention provides a redox curing dental composition wherein a compound of formula (III) is contained in either of the first or the second paste.

The invention will now be further illustrated by the following Examples.

### Examples

### Materials and methods

### Chemical Compounds

All the reactants were selected with high purity and used as received (Chart 1). Methyldiphenylsilane (MDPS), Dimethylphenylsilane (DMPS), Triphenylsilane (TPS), Methyltriphenylsilane (MTPS) and Methoxydimethylphenylsilane (MODMPS) were purchased from Alfa Aesar. Diphenylsilane (DPS) (purity>98%), 2-(Diphenylphosphino)benzoic Acid (2DPPBA) and 4-(Dimethylamino)phenyldiphenylphosphine (DMAPDP) were purchased from TCI. Diphenylsilane (DPS) (purity>97%), 4-(Diphenylphosphino)styrene (4DPPS) were purchased from Sigma-Aldrich. Di-tert-butyl-diphenyl iodonium hexafluorophosphate (Iod) was obtained from Lambson Ltd (UK).

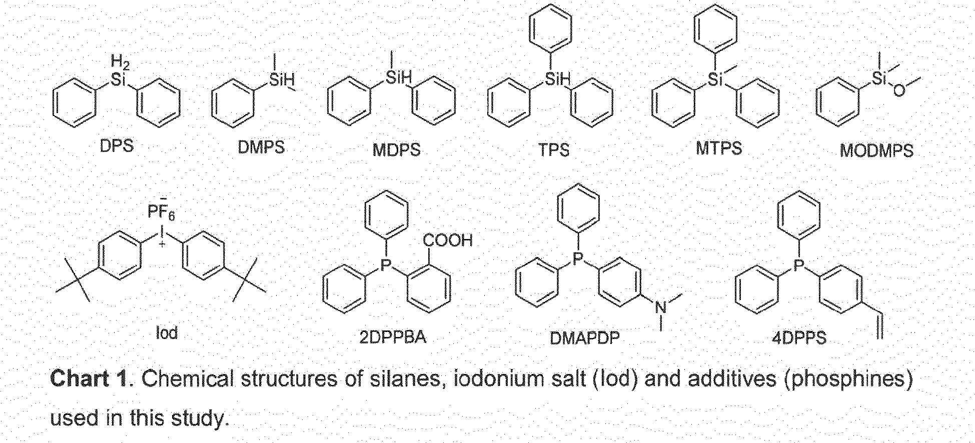

The efficiency of different silane/lod and silane/lod/phosphine initiating systems were checked by using different (meth)acrylate monomers or blends; denoted as **Resin 1**: bisGMA-TEGDMA (bisphenol A-glycidyl methacrylate and triethylene glycol dimethacrylate) was composed of a bisGMA/TEGDMA blend (70/30% w/w) obtained from Sigma-Aldrich; **Resin 2**: a model methacrylate resin was composed of UDMA (urethane dimethacrylate) /HPMA (hydroxypropyl methacrylate)/BDDMA(butanediol dimethacrylate) (33.3/33.3/33.3 % w/w/w); **Resin 3**: a blend was composed of a UDMA/PEG200DMA/BDDMA (33.3/33.3/33.3% w/w/w); **Resin 4**: Ebecryl 605 (acrylate resin) was composed of a epoxyacrylate/tripropyleneglycoldiacrylate (75/25% w/w). All formulations (Chart 2) were prepared from the monomers from Sigma-Aldrich (except Ebecryl 605 from Cytec) and redox polymerizations were carried out at room temperature (RT) (21-25 °C) under air.

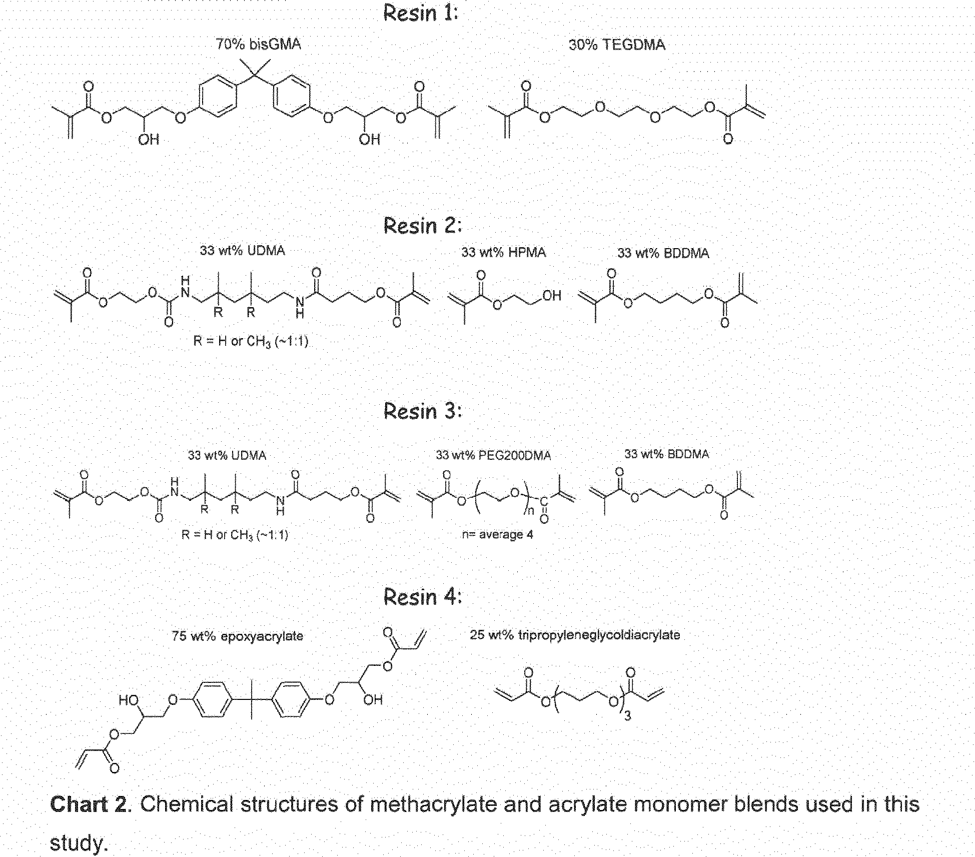

### Definition of the Two Components Used for Redox Experiments

All redox formulations were prepared from the bulk resins in two separate cartridges: a first cartridge (cartridge 1) with the silane and the other one (cartridge 2) containing the iodonium salt (Iod) (Figure 1B). A 1:1 Sulzer mixpac mixer was used to mix all components together at the beginning of each polymerization experiment. All polymerization experiments will be performed under mild conditions: at room temperature (RT) (21-23 °C) and under air.

### Definition of the Three Components Used for Redox Experiments

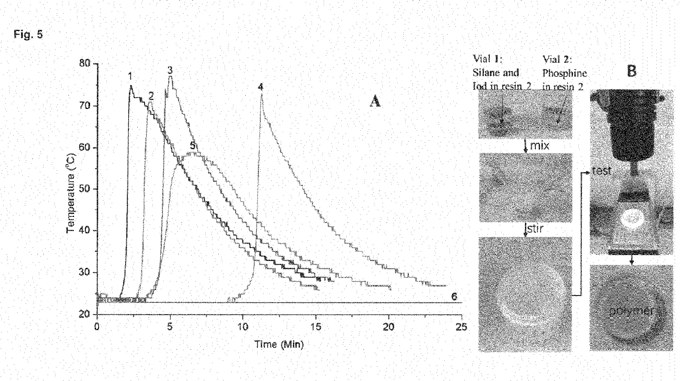

All redox formulations were prepared from the bulk resins in two separate vials: a first vial (vial 1) with the silane and lod, the other one (vial 2) containing the phosphine (Figure 5B).

Then, the same volume of formulation from vial 1 and vial 2 was transferred to a plastic cup at the same time and was stirred about 10 s quickly at the beginning of each polymerization experiment. All polymerization experiments will be performed under mild conditions: at room temperature (RT) (21-23 °C) and under air.

### Polymerization in Bulk Followed by Optical Pyrometry

The use of optical pyrometry to follow polymerization reactions was used here: temperature versus time profiles were followed using an Omega OS552-V1-6 infrared thermometer (Omega Engineering, Inc., Stamford, CT) having a sensitivity of ±1 °C for 2 g (sample thickness∼4 mm). This setup was also used to monitor the T (°C) vs time upon irradiation with a LED@405 nm.

### Differential Scanning Calorimetry (DSC) for Thermal Polymerization

10 mg of fresh formulations in presence of RIS were inserted in an aluminum 100 µL crucible. Thermal polymerization was carried out from 0 °C to 300 °C at a heating rate of 10 °C/min under nitrogen flow (100 mL/min). A METTLER-TOLEDO DSC differential scanning calorimeter was used.

### Preparation of Composites

Sulzer mixpac mixer was used to inject the fresh formulation in presence of RIS onto the carbon or glass fibers fabrics. The ratio of the resin to the fabric was controlled at about 50/50% (w/w) by a balance. After a few minutes, the fabric was completely impregnated by the resin formulation, and the prepregs started the curing process at room temperature, under air.

### Electron Spin Resonance (ESR) Spin Trapping (ESR-ST)

Electron spin resonance-spin trapping experiments were carried out using an X-band spectrometer (Magnettech MS400). The radicals were observed under nitrogen saturated media at room temperature. N-tert-butyl nitrone (PBN) was used as a spin trap agent in tert-butylbenzene. ESR spectra simulations were carried out using WINSIM software.

### Results and discussions

Optical pyrometric measurements were chosen as they offer a fast and reliable assessment of polymerization efficiency (through its exothermicity). From the curves (Figure 1), it can be seen that DPS/lod redox initiator system (RIS) exhibited outstanding initiating property with a fast polymerization (gel time = 1.5 min corresponding to the time needed to reach the maximal temperature). Remarkably, combination of DPS and lod leads to a high exothermicity of 105 °C i.e. similar to amine/benzoylperoxide (100 °C) using the same resin and setup. Therefore, it can be concluded that the new RIS is at least as efficient as this reference. DMPS/lod and MDPS/lod RIS exhibited a much lower activity than DPS/lod (same resin) i.e. after 2.5 h mixing, a solid was found at the bottom of the beaker with a liquid layer on top of it. On the contrary, for TPS/lod, MTPS/lod and MODMPS/lod RIS resin system, after 24 h, no polymerization at all was observed. No curing of the resin 1 was obtained using DPS or lod alone showing that the 2K mixing of these compounds is clearly needed to trigger the DPS/lod redox reaction.

In redox initiated free radical polymerization (RFRP), as in most of polymerization initiations, efficient initiating systems need to produce high enough initiating radicals per unit of time to counter inhibiting reactions (e.g. dynamic oxygen inhibition under air and phenolic stabilizers).

Here, the low initiating activity of DMPS and MDPS may be ascribed to its higher steric hindrance on the Si-H functionality compared to DPS. For TPS the steric hindrance of the silane is quite high. Therefore, it is probably hard for lod to be close enough to the Si-H bond (free radical cannot be generated). For MTPS and MODMPS, there is no hydrogen atom in the silane structure; accordingly no reduction reaction can occur i.e. free radicals cannot be generated.

In order to optimize the material preparation and the reaction times, concentration of RIS has to be assessed. Indeed, gel times of 2-10 min are often required in the industry in order to have sufficient workability. For example, the 2K mixed resin has to have sufficient time to soak the fibers or to fill the mold and then rapidly cure, avoiding premature curing during the first 1-2 minutes of the mix.

The polymerization profiles of the methacrylates (Resin 1) under air are depicted in Figure 2. As shown, the high-efficiency RIS developed here for room temperature exactly meets this requirement, and the curing time is controlled within 2-15 min. There is a positive effect of an increase of the content of silane and lod to decrease the gel time (i.e. when more DPS and iodonium salt was added, a short time of polymerization is expected). The polymerization process is very fast if the concentration of the DPS and lod are higher than 0.8 wt % (maximum temperature T > 100 °C; starting reaction time < 1 min; Figure 2, curve 6, 7). Interestingly, the process is quite efficient if the concentration of the silane and lod is higher than 0.2 wt % (maximum temperature T > 50 °C; Figure 2, curve 2, 3, 4, 5, 8, 9). Therefore, it has high potential to control the gel time if the concentration of the DPS and lod was tuned between 0.2 wt% and 0.8 wt%. The data of polymerization based on different concentration of DPS and lod are summarized in Table 1; excellent tuning of the reaction times (up to 15 minutes) is made possible.

**Table 1. Summarized Data of Polymerization based on Different Concentration of DPS and lod for Resin 1.**

| DPS concentration (wt%) | Iod concentration (wt%) | Reaction starting time (min) | Max. Temperature peak time (min) | Peak temperature (°C) |
|---|---|---|---|---|
| 0.1 | 0.1 | >25 | - | - |
| 0.2 | 0.2 | 15 | 18 | 53 |
| 0.3 | 0.3 | 5.5 | 8 | 63 |
| 0.4 | 0.4 | 3 | 4.2 | 74 |
| 0.5 | 0.5 | 2.7 | 3.1 | 109 |
| 0.8 | 0.8 | 1.3 | 1.6 | 106 |
| 1.0 | 1.0 | 1.3 | 1.5 | 104 |
| 0.2 | 0.4 | 6 | 7.1 | 74 |
| 0.4 | 0.2 | 6.5 | 7.8 | 73 |

In order to certify the role of the silane, the DPS from other supplier (TCI) was compared to that of from Sigma-Aldrich. A same perfect initiating efficiency was obtained, indicating the robustness of newly developed DPS/lod system. At the same time, in order to examine whether or not the initiator is sensitive to the light, the polymerization profiles with and without irradiation have been compared. The results showed that this kind of RIS is not sensitive to light.

Furthermore, the initiating ability of DPS/lod RIS for other (meth)acrylate resins was checked, as shown in Figure 3. The RIS exhibited excellent initiating ability of i) ebecryl resin (Resin 4), ii) the mixing resin of of Resin 1 and 2 or iii) Resin 3 with Resin 1. For the mixing resin, the polymerization finished within only 5 min in presence of 0.5 wt% RIS. Interestingly, for Resin 4, it is possible to have comfortable gel times of more than 35 minutes (Figure 3, curve 1) with excellent efficiency (120 °C exothermicity) which is not possible with amine/peroxide reference RIS under air at room temperature.

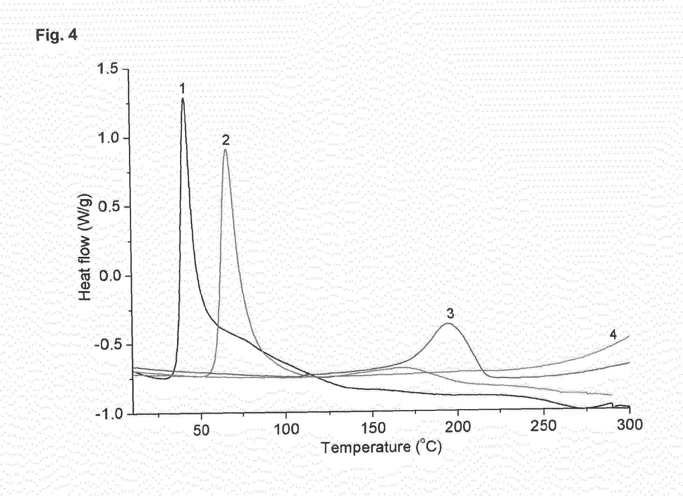

In order to: i) exclude thermal decomposition of separate lod and DPS and ii) show potential thermal B-stage curing; the initiating ability of DPS/Iod RIS upon heating by DSC experiment right after 2K mixing (Figure 4) was explored. The results showed that DPS/Iod resin system was very sensitive to heat even for a relatively low initiator concentration. For the system with a 0.2 wt% RIS concentration, the polymerization process started at 25 °C, which is consistent with the optical pyrometry results (polymerizations without heating). For the system with a 0.1 wt% RIS concentration, the polymerization process started at 52 °C. For the sample containing DPS alone, the polymerization began when the resin was heated above 150 °C (and with poor efficiency). For the system containing lod alone, the polymerization did not occur bellow 300 °C showing clearly that the presence of both DPS and lod is required for curing at low temperature. Therefore, DSC results further indicated the synergic initiating effect of DPS and lod in a redox reaction.

Next step, the initiating efficiency of DPS/Iod in presence of different phosphines as additives was investigated. Especially, Resin 2 was selected as it has a very low viscosity (0.05 Pa.s) which leads to extremely high oxygen inhibition. The different polymerization profiles in the presence of phosphine additives are reported in Figure 5.

It can be seen that the RIS with DMAPDP (Chart 1) as an additive exhibited highest initiating efficiency (with only 1.0 wt% concentration, the polymerization reaction started within 2 min, Figure 5, curve 1). Remarkably, in Resin 2 (a resin that is difficult to polymerize due to low viscosity), the three-component RIS exhibited excellent initiating ability. RIS with 2DPPBA or 4DPPS as an additive also exhibited perfect initiating efficiency (with 2.0 wt% concentration, the polymerization reaction started within 5 min, Figure 5, curve 3 and 5). There is a positive effect of decreasing the content of silane and lod to extend the induction time (i.e. when less DPS, lod and phosphine was added, a longer time is expected for the polymerization). Therefore, the proposed upgraded DPS/lod/phosphine RIS can be considered as a good way to cure radical monomers upon mild room temperature conditions.

The initiating ability of DPS/Iod/phosphine RIS upon heating was also investigated by DSC experiment (Figure 6). The results showed that DPS/Iod/phosphine thermal initiator and resin system was very sensitive to heat. For the system with a 1.0 wt% DPS/Iod/2DPPBA RIS concentration, the polymerization process started below 40 °C, which is consistent to the optical pyrometric measurement result (heat not required). For the system contained DPS/Iod without additive (in Resin 2), the polymerization began when the resin was heated near 100 °C, and polymerization is very intensive. Therefore, DSC results further indicated the synergic initiating effect of DPS, lod and phosphine at a mild temperature even for a low reactivity monomer blend (Resin 2).

The main advantages of composite materials are their high mechanical properties, relatively low weight and corrosion resistance. Indeed, smart combination of fibers (or fillers) and polymeric resins can be performed. Here, the new proposed DPS/Iod system was used as a RIS for the synthesis of methacrylate/glass fibers or methacrylate/carbon fibers composites at room temperature. From the results of optical pyrometric measurements, the initiator concentration of 0.4 wt% DPS/0.4 wt% Iod RIS and 0.2 wt% DPS/0.4 wt% lod was chosen to prepare composites (i.e. efficient systems but with an adapted gel time to ensure a good impregnation of the fibers). With the selected inducing time (3min and 6 min), this allows the resin to be soaked fully into the fibers. First of all, glass fibers or carbon fibers were impregnated by methacrylate resin embedded with RIS. After about 5-10 min, the obtained prepregs (Figure 7: F1-F4) became solid. Interestingly, the presence of glass fibers or carbon fibers does not delay the gel time of free radical polymerization. The surface and bottom of the composites looked a little tacky (probably due to the oxygen inhibition). However, after they were heated about 1 h at 100 °C under air, the small residue of starchy resin became fully solid (Figure 7: C1-C4). Here, one can see the usefulness of this system being able to cure through thermal B-stage (Figure 4). Also, the robustness of redox polymerization enables access to carbon fibers composites which is generally not possible using light induced polymerizations.

Next, the preparation of composites was performed using the highly robust three-component DPS/Iod/phosphine RIS for the synthesis of methacrylate/glass fibers and methacrylate/carbon fibers composites at room temperature. From the results of optical pyrometric measurements, initiator concentration of 0.5 wt% DPS/0.5 wt% lod/0.5 wt% DMAPDP and 1.0 wt% DPS/1.0 wt% lod/1.0 wt% 2DPPBA were selected to prepare composites. First of all, glass fibers and carbon fibers were impregnated by fresh formulations in presence of three-component RIS, prepregs were obtained (Figure 8: F1-F4). For DPS/lod/DMAPDP resin system, after about 5 min, the composite became solid. For carbon fibers reinforced composites, both surface and bottom are tacky-free (Figure 8: C1-C2). It is a perfect method to prepare carbon fibers composites at room temperature using DPS/lod/DMAPDP as FRP initiator. For DPS/lod/2DPPBA resin system, after about 90 min fibers impregnation by the resin, the system became solid (Figure 8: C3-C4).

For a better understanding of the DPS/Iod interaction, ESR-spin trapping experiments were carried out (Figure 9). In presence of PBN as spin trap agent, some radicals are directly detected after mixing of DPS and lod in tert-butylbenzene under N₂. Two PBN/radical adducts are detected: PBN/Ar^{•} characterized by *α*_{N} = 14.1G; *α*_{N} = 2.1G and PBN/H^{•} characterized by *α*_{N} = 14.5G; *α*_{N} (2) = 7.1G in agreement with literature data. This suggests the redox reaction (r1) between DPS and lod as the iodonium salt is reduced leading to aryl radicals.

DPS + Ar₂I⁺ →→ Ar^{•} + H^{•} (r1)

The radicals generated in r1 can be assumed as the initiating species for the FRP processes observed above.

Figure 10 shows that besides suitable iodonium based oxidizing agents also suitable metal cation based oxidizing agents can be used in the redox initiator system according to the present invention. In the Example shown in Fig. 10 copper (II) methacryloxyethylacetoacetate was used as an oxidizing agent and it was shown that with this system also a suitable gel time can be achieved.

The photopolymerization initiating efficiency of DPS/Iod was firstly measured by optical pyrometric measurements (Figure 11A) and RT-FTIR (Figure 11B). As shown in Figure 11, DPS combined with lod possess a very low ability to initiate a visible light polymerization (due to the poor light absorption properties of DPS and lod - absorbing significantly only for □ < 350 nm). When Camphorquinone (CQ) is added, the initiating efficiency increased significantly. The conversion rate of resin 2 reached 85%. Then, the monomer conversion rate at different distance from the surface was measured by Raman spectroscopy for the DPS/Iod, DPS/CQ and DPS/Iod/CQ system. For CQ/DPS/Iod system, the conversion rate throughout the entire polymer increased from 20% at the surface to 90% in the bulk; interestingly, the thickness of the oxygen-inhibited layer decreased to a quite thin layer. All the test results showed that the CQ/DPS/Iod system exhibits excellent photoinitiation properties. As known for CQ that is a Type II photoinitiator, this performance can be ascribed to r2-r5 which lead to additional initiating radicals than the redox reaction in DPS/lod. The reactions r2-r5 were shown for other Type II photoinitiators in "Photoinitiators for Polymer Synthesis", Wiley, Weinheim 2012, J. Lalevée, J.P. Fouassier.

CQ (h□) → *CQ

*CQ + Ph₂SiH₂ → CQ-H^{•} + Ph₂Si^{•}H r2

*CQ + Ar₂I⁺ → CQ^{•+} + Ar^{•} + ArI r3

CQ-H^{•} + Ar₂I⁺ → CQ + H⁺ + Ar^{•} + ArI r4

Ph₂Si^{•}H + Ar₂I⁺ → Ph₂Si⁺H + Ar^{•} + ArI r5

## Claims

1. A redox curing dental composition comprising:
(a) one or more compounds having a radical polymerizable carbon-carbon double bond;
(b) a redox initiator system comprising
(i) a reducing agent which is a compound of the following formula (I): 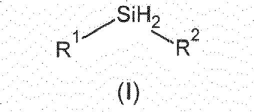 wherein
R¹ and R²
which may be the same or different, independently represent a C₁₋₁₈ hydrocarbyl group, or R¹ and R² form together with the silicon atom to which they are bonded a 3 to 8-membered silicon-containing alicyclic ring, which groups R¹ and/or R² may be substituted with one to three substituents selected from the group consisting of a C₁₋₆ alkoxy group, a halogen atom, or a carboxylic acid group; and
(ii) an oxidizing agent adapted to oxidize the reducing agent of formula (I) in the absence of light for generating a radical capable of initiating radical polymerization of the one or more compounds having a radical polymerizable carbon-carbon double bond.

2. The redox curing dental composition according to claim 1, wherein the oxidizing agent has a reduction potential in the range of from -0.15 to -1.2 V as determined by cyclic voltammetry.

3. The redox curing dental composition according to claim 1 or 2, wherein the oxidizing agent has a storage stability so that a 1 mol/liter aqueous solution of the oxidizing agent decomposes in an amount of less than 10 % when stored at 50 °C for four weeks.

4. The redox curing dental composition according to any one of the preceding claims, wherein the oxidizing agent contains a metal cation or a cation of the following formula (II): 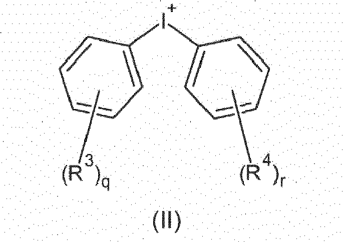 wherein
R³ which may be the same or different when more than one R³ is present, independently represents a C₁₋₆ alkyl group, C₂₋₆ alkenyl group, a C₁₋₆ alkoxy group, a halogen atom, or a carboxylic acid group;
R⁴ which may be the same or different when more than one R⁴ is present, independently represents, a C₁₋₆ alkyl group, C₂₋₆ alkenyl group, a C₁₋₆ alkoxy group, a halogen atom, or a carboxylic acid group; or
a substituent R³ and a substituent R⁴ form together a single bond or a C₁₋₆ alkylene or C₂₋₆ alkenylene group;
q and r, which may be the same or different, represent 0 or an integer of 1 to 5.

5. The redox curing dental composition according to any one of the preceding claims, wherein the redox initiator system further comprises a phosphine compound of the following formula (III): 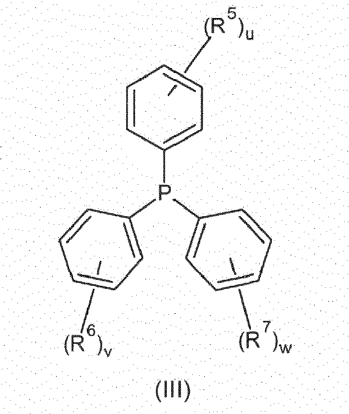 wherein
R⁵ which may be the same or different when more than one R⁵ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group;
R⁶ which may be the same or different when more than one R⁶ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group;
R⁷ which may be the same or different when more than one R⁷ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group; or
two substituents selected from R⁵, R⁶ and R⁷ form together a single bond or a C₁₋₆ alkylene or C₂₋₆ alkenylene group;
u, v and w, which may be the same or different, represent 0 or an integer of 1 to 5.

6. The redox curing dental composition according to any one of the preceding claims, wherein R¹ and R² which may be the same or different, independently represent an unsubstituted C₁₋₆ alkyl group, or an unsubstituted C₆₋₁₆ aryl group.

7. The redox curing dental composition according to any one of the preceding claims, wherein q and r represent 1, and R³ and R⁴ independently represent a C₁₋₆ alkyl group.

8. The redox curing dental composition according to any one of claims 5 to 7, wherein u+v+w=1.

9. The redox curing dental composition according to any one of the preceding claims, which further comprises a photosensitizer, preferably having an absorption maximum in the range of 400 to 800 nm.

10. The redox curing dental composition according to any one of the preceding claims, which further comprises a particulate filler.

11. The redox curing dental composition according to any one of the preceding claims, which is selected from the group of a resin-modified dental cement, and a dental bonding agent.

12. The redox curing dental composition according to any one of the preceding claims, which is a two part paste-paste composition comprising a first paste containing the reducing agent (i), and a second paste containing the oxidizing agent (ii).

13. A redox initiator system comprising
(i) a reducing agent which is a compound of the following formula (I): 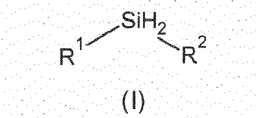 wherein
R¹ and R²
which may be the same or different, independently represent a C₁₋₁₈ hydrocarbyl group, or R¹ and R² form together with the silicon atom to which they are bonded a 3 to 8-membered silicon-containing alicyclic ring, which groups R¹ and/or R² substituted with one to three substituents selected from the group consisting of a C₁₋₆ alkoxy group, a halogen atom, or a carboxylic acid group; and
(ii) an oxidizing agent adapted to oxidize the reducing agent of formula (I) in the absence of light for generating a radical capable of initiating radical polymerization of a one or more compounds having a radical polymerizable carbon-carbon double bond.

14. The redox initiator system according to claim 13, wherein the redox initiator system further comprises ap phosphine compound of the following formula (III): 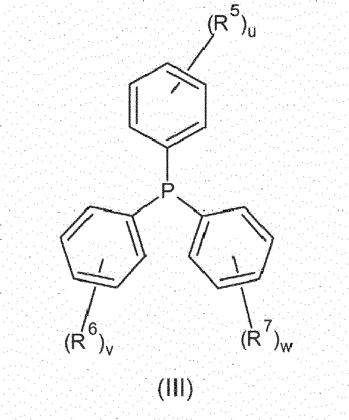 wherein
R⁵ which may be the same or different when more than one R⁵ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group;
R⁶ which may be the same or different when more than one R⁶ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group;
R⁷ which may be the same or different when more than one R⁷ is present, independently represents a C₁₋₆ alkyl group, C₂₋₆ alkenyl group, a C₁₋₆ alkoxy group, a C₁₋₆ alkylamino group, a di-(C₁₋₆ alkyl)amino group a halogen atom, or a carboxylic acid group; or
two substituents selected from R⁵, R⁶ and R⁷ form together a single bond or a C₁₋₆ alkylene or C₂₋₆ alkenylene group;
u, v and w, which may be the same or different, represent 0 or an integer of 1 to 5.

15. The redox initiator system according to claims 13 or 14 for use in the treatment or prevention of endodontic disease.
